# EUROPEAN PATENT APPLICATION

(11) **EP 2 801 372 A2**
(43) Date of publication of application: **12.11.2014**
(21) Application number: 14167980.3
(22) Date of filing: 12.05.2014
(51) Int. Cl.: A61K 39/145, C07K 14/005

(54) **Avoiding narcolepsy risk in influenza vaccines**

(30) Priority: 10.05.2013 US 201361822228 P; 26.07.2013 US 201361859113 P; 06.08.2013 US 201361862807 P; 23.08.2013 EP 13181429; 11.03.2014 EP 14158999
(71) Applicant: Novartis AG, 4056 Basel (CH); Volkmuth, Wayne, Foster City, CA 94404 (US); Steinman, Lawrence, Stanford, CA 94305 (US)
(72) Inventor: Volkmuth, Wayne, Foster City, CA California 94404 (US); Steinman, Lawrence, Stanford, CA California 94305 (US); Ahmed, Syed Sohail, 53100 Siena (IT)
(74) Representative: Carpmaels & Ransford LLP

(57) **Abstract**

The invention provides influenza vaccines and methods which improve the safety of influenza vaccines further, in particular in relation to the risk of causing narcolepsy in adjuvanted vaccines.

SEQUENCE LISTING

<110>   NOVARTIS AG
        Lawrence STEINMAN
        Wayne VOLKMUTH

<120>   AVOIDING NARCOLEPSY RISK IN INFLUENZA VACCINES

<130>   P063804WO

<140>   PCT/EP2014/______
<141>   2014-05-09

<150>   US 61/822,228
<151>   2013-05-10

<150>   US 61/859,113
<151>   2013-07-26

<150>   US 61/862,807
<151>   2013-08-06

<150>   EP-13181429.5
<151>   2013-08-23

<150>   EP-14158999.4
<151>   2014-03-11

<160>   33

<170>   SeqWin2010, version 1.0

<210>   1
<211>   21
<212>   PRT
<213>   Influenza A virus

<400>   1
Arg Glu Leu Ile Leu Tyr Asp Lys Glu Glu Ile Arg Arg Ile Trp Arg
1               5                   10                  15

Gln Ala Asn Asn Gly
            20

<210>   2
<211>   498
<212>   PRT
<213>   Influenza A virus

<400>   2
Met Ala Ser Gln Gly Thr Lys Arg Ser Tyr Glu Gln Met Glu Thr Asp
1               5                   10                  15

Gly Glu Arg Gln Asn Ala Thr Glu Ile Arg Ala Ser Val Gly Lys Met
            20                  25                  30

Ile Gly Gly Ile Gly Arg Phe Tyr Ile Gln Met Cys Thr Glu Leu Lys
        35                  40                  45

Leu Ser Asp Tyr Glu Gly Arg Leu Ile Gln Asn Ser Leu Thr Ile Glu
    50                  55                  60

Arg Met Val Leu Ser Ala Phe Asp Glu Arg Arg Asn Lys Tyr Leu Glu

65                  70                  75                  80

Glu His Pro Ser Ala Gly Lys Asp Pro Lys Lys Thr Gly Gly Pro Ile
                85                  90                  95

Tyr Arg Arg Val Asn Gly Lys Trp Met Arg Glu Leu Ile Leu Tyr Asp
            100                 105                 110

Lys Glu Glu Ile Arg Arg Ile Trp Arg Gln Ala Asn Asn Gly Asp Asp
        115                 120                 125

Ala Ala Ala Gly Leu Thr His Met Met Ile Trp His Ser Asn Leu Asn
    130                 135                 140

Asp Ala Thr Tyr Gln Arg Thr Arg Ala Leu Val Arg Thr Gly Met Asp
145                 150                 155                 160

Pro Arg Met Cys Ser Leu Met Gln Gly Ser Thr Leu Pro Arg Arg Ser
                165                 170                 175

Gly Ala Ala Gly Ala Ala Val Lys Gly Val Gly Thr Met Val Met Glu
            180                 185                 190

Leu Val Arg Met Ile Lys Arg Gly Ile Asn Asp Arg Asn Phe Trp Arg
        195                 200                 205

Gly Glu Asn Gly Arg Lys Thr Arg Ile Ala Tyr Glu Arg Met Cys Asn
    210                 215                 220

Ile Leu Lys Gly Lys Phe Gln Thr Ala Ala Gln Lys Ala Met Met Asp
225                 230                 235                 240

Gln Val Arg Glu Ser Arg Asn Pro Gly Asn Ala Glu Phe Glu Asp Leu
                245                 250                 255

Thr Phe Leu Ala Arg Ser Ala Leu Ile Leu Arg Gly Ser Val Ala His
            260                 265                 270

Lys Ser Cys Leu Pro Ala Cys Val Tyr Gly Pro Ala Val Ala Ser Gly
        275                 280                 285

Tyr Asp Phe Glu Arg Glu Gly Tyr Ser Leu Val Gly Ile Asp Pro Phe
    290                 295                 300

Arg Leu Leu Gln Asn Ser Gln Val Tyr Ser Leu Ile Arg Pro Asn Glu
305                 310                 315                 320

Asn Pro Ala His Lys Ser Gln Leu Val Trp Met Ala Cys His Ser Ala
                325                 330                 335

Ala Phe Glu Asp Leu Arg Val Leu Ser Phe Ile Lys Gly Thr Lys Val
            340                 345                 350

Leu Pro Arg Gly Lys Leu Ser Thr Arg Gly Val Gln Ile Ala Ser Asn
        355                 360                 365

Glu Asn Met Glu Thr Met Glu Ser Ser Thr Leu Glu Leu Arg Ser Arg
    370                 375                 380

Tyr Trp Ala Ile Arg Thr Arg Ser Gly Gly Asn Thr Asn Gln Gln Arg
385                 390                 395                 400

Ala Ser Ala Gly Gln Ile Ser Ile Gln Pro Thr Phe Ser Val Gln Arg

405                 410                 415

Asn Leu Pro Phe Asp Arg Thr Thr Ile Met Ala Ala Phe Asn Gly Asn
            420                 425                 430

Thr Glu Gly Arg Thr Ser Asp Met Arg Thr Glu Ile Ile Arg Met Met
        435                 440                 445

Glu Ser Ala Arg Pro Glu Asp Val Ser Phe Gln Gly Arg Gly Val Phe
    450                 455                 460

Glu Leu Ser Asp Glu Lys Ala Ala Ser Pro Ile Val Pro Ser Phe Asp
465                 470                 475                 480

Met Ser Asn Glu Gly Ser Tyr Phe Phe Gly Asp Asn Ala Glu Glu Tyr
                485                 490                 495

Asp Asn

<210>   3
<211>   21
<212>   PRT
<213>   Influenza A virus

<400>   3
Arg Glu Leu Ile Leu Tyr Asp Lys Glu Glu Met Arg Arg Ile Trp Arg
1               5                   10                  15

Gln Ala Asn Asn Gly
            20

<210>   4
<211>   21
<212>   PRT
<213>   Influenza A virus

<400>   4
Trp Arg Gln Ala Asn Asn Gly Asp Asp Ala Ala Ala Gly Leu Thr His
1               5                   10                  15

Met Met Ile Trp His
            20

<210>   5
<211>   21
<212>   PRT
<213>   Influenza A virus

<400>   5
Trp Arg Gln Ala Asn Asn Gly Asp Asp Ala Thr Ala Gly Leu Thr His
1               5                   10                  15

Met Met Ile Trp His
            20

<210>   6
<211>   21
<212>   PRT
<213>   Influenza A virus

<400>   6
Lys Thr Ser Ser Trp Pro Asn His Asp Ser Asn Lys Gly Val Thr Ala

1                   5                   10                  15

Ala Cys Pro His Ala
            20

<210>   7
<211>   21
<212>   PRT
<213>   Influenza A virus

<400>   7
Lys Thr Ser Ser Trp Pro Asn His Asp Ser Asp Lys Gly Val Thr Ala
1                   5                   10                  15

Ala Cys Pro His Ala
            20

<210>   8
<211>   9
<212>   PRT
<213>   Influenza A virus

<400>   8
Leu Ile Leu Tyr Asp Lys Glu Glu Ile
1                   5

<210>   9
<211>   14
<212>   PRT
<213>   Influenza A virus

<220>
<221>   misc_feature
<222>   2, 5, 6, 7, 9, 10, 11, 12, 13, 14
<223>   'Xaa' is any amino acid

<400>   9
Leu Xaa Leu Tyr Xaa Xaa Xaa Ile Xaa Xaa Xaa Xaa Xaa Xaa
1                   5                   10

<210>   10
<211>   19
<212>   PRT
<213>   Influenza A virus

<400>   10
Leu Ile Leu Tyr Asp Lys Glu Glu Ile Arg Arg Ile Trp Arg Gln Ala
1                   5                   10                  15

Asn Asn Gly

<210>   11
<211>   9
<212>   PRT
<213>   Influenza A virus

<220>
<221>   misc_feature
<222>   9
<223>   'Xaa' is any amino acid

<400>   11

Leu Ile Leu Tyr Asp Lys Glu Glu Xaa
1               5

<210>   12
<211>   498
<212>   PRT
<213>   Influenza A virus

<400>   12
Met Ala Ser Gln Gly Thr Lys Arg Ser Tyr Glu Gln Met Glu Thr Asp
1               5                   10                  15

Gly Glu Arg Gln Asn Ala Thr Glu Ile Arg Ala Ser Val Gly Lys Met
            20                  25                  30

Ile Gly Gly Ile Gly Arg Phe Tyr Ile Gln Met Cys Thr Glu Leu Lys
        35                  40                  45

Leu Ser Asp Tyr Glu Gly Arg Leu Ile Gln Asn Ser Leu Thr Ile Glu
    50                  55                  60

Arg Met Val Leu Ser Ala Phe Asp Glu Arg Arg Asn Lys Tyr Leu Glu
65                  70                  75                  80

Glu His Pro Ser Ala Gly Lys Asp Pro Lys Lys Thr Gly Gly Pro Ile
                85                  90                  95

Tyr Arg Arg Val Asn Gly Lys Trp Met Arg Glu Leu Ile Leu Tyr Asp
            100                 105                 110

Lys Glu Glu Met Arg Arg Ile Trp Arg Gln Ala Asn Asn Gly Asp Asp
        115                 120                 125

Ala Thr Ala Gly Leu Thr His Met Met Ile Trp His Ser Asn Leu Asn
    130                 135                 140

Asp Ala Thr Tyr Gln Arg Thr Arg Ala Leu Val Arg Thr Gly Met Asp
145                 150                 155                 160

Pro Arg Met Cys Ser Leu Met Gln Gly Ser Thr Leu Pro Arg Arg Ser
                165                 170                 175

Gly Ala Ala Gly Ala Ala Val Lys Gly Val Gly Thr Met Val Met Glu
            180                 185                 190

Leu Val Arg Met Ile Lys Arg Gly Ile Asn Asp Arg Asn Phe Trp Arg
        195                 200                 205

Gly Glu Asn Gly Arg Lys Thr Arg Ile Ala Tyr Glu Arg Met Cys Asn
    210                 215                 220

Ile Leu Lys Gly Lys Phe Gln Thr Ala Ala Gln Lys Ala Met Met Asp
225                 230                 235                 240

Gln Val Arg Glu Ser Arg Asn Pro Gly Asn Ala Glu Phe Glu Asp Leu
                245                 250                 255

Thr Phe Leu Ala Arg Ser Ala Leu Ile Leu Arg Gly Ser Val Ala His
            260                 265                 270

Lys Ser Cys Leu Pro Ala Cys Val Tyr Gly Pro Ala Val Ala Ser Gly
        275                 280                 285

Tyr Asp Phe Glu Arg Glu Gly Tyr Ser Leu Val Gly Ile Asp Pro Phe
    290                 295                 300

Arg Leu Leu Gln Asn Ser Gln Val Tyr Ser Leu Ile Arg Pro Asn Glu
305                 310                 315                 320

Asn Pro Ala His Lys Ser Gln Leu Val Trp Met Ala Cys His Ser Ala
                325                 330                 335

Ala Phe Glu Asp Leu Arg Val Leu Ser Phe Ile Lys Gly Thr Lys Val
            340                 345                 350

Leu Pro Arg Gly Lys Leu Ser Thr Arg Gly Val Gln Ile Ala Ser Asn
        355                 360                 365

Glu Asn Met Glu Thr Met Glu Ser Ser Thr Leu Glu Leu Arg Ser Arg
    370                 375                 380

Tyr Trp Ala Ile Arg Thr Arg Ser Gly Gly Asn Thr Asn Gln Gln Arg
385                 390                 395                 400

Ala Ser Ala Gly Gln Ile Ser Ile Gln Pro Thr Phe Ser Val Gln Arg
                405                 410                 415

Asn Leu Pro Phe Asp Arg Thr Thr Ile Met Ala Ala Phe Asn Gly Asn
            420                 425                 430

Thr Glu Gly Arg Thr Ser Asp Met Arg Thr Glu Ile Ile Arg Met Met
        435                 440                 445

Glu Ser Ala Arg Pro Glu Asp Val Ser Phe Gln Gly Arg Gly Val Phe
    450                 455                 460

Glu Leu Ser Asp Glu Lys Ala Ala Ser Pro Ile Val Pro Ser Phe Asp
465                 470                 475                 480

Met Ser Asn Glu Gly Ser Tyr Phe Phe Gly Asp Asn Ala Glu Glu Tyr
                485                 490                 495

Asp Asn


<210>   13
<211>   498
<212>   PRT
<213>   Influenza A virus

<400>   13


<210>   14
<211>   120
<212>   PRT
<213>   Homo sapiens

<400>   14
Thr Lys Leu Glu Asp Ser Pro Pro Cys Arg Asn Trp Ser Ser Ala Ser
1               5                   10                  15

Glu Leu Asn Glu Thr Gln Glu Pro Phe Leu Asn Pro Thr Asp Tyr Asp
            20                  25                  30

Asp Glu Glu Phe Leu Arg Tyr Leu Trp Arg Glu Tyr Leu His Pro Lys
        35                  40                  45

Glu Tyr Glu Trp Val Leu Ile Ala Gly Tyr Ile Ile Val Phe Val Val
    50                  55                  60

Ala Leu Ile Gly Asn Val Leu Val Cys Val Ala Val Trp Lys Asn His
65                  70                  75                  80

His Met Arg Thr Val Thr Asn Tyr Phe Ile Val Asn Leu Ser Leu Ala
                85                  90                  95

Asp Val Leu Val Thr Ile Thr Cys Leu Pro Ala Thr Leu Val Val Asp
            100                 105                 110

Ile Thr Glu Thr Trp Phe Phe Gly
        115                 120

<210>   15
<211>   120
<212>   PRT
<213>   Homo sapiens

<400>   15
Met Glu Pro Ser Ala Thr Pro Gly Ala Gln Met Gly Val Pro Pro Gly
1               5                   10                  15

Ser Arg Glu Pro Ser Pro Val Pro Pro Asp Tyr Glu Asp Glu Phe Leu
            20                  25                  30

Arg Tyr Leu Trp Arg Asp Tyr Leu Tyr Pro Lys Gln Tyr Glu Trp Val
        35                  40                  45

Leu Ile Ala Ala Tyr Val Ala Val Phe Val Val Ala Leu Val Gly Asn
    50                  55                  60

Thr Leu Val Cys Leu Ala Val Trp Arg Asn His His Met Arg Thr Val
65                  70                  75                  80

Thr Asn Tyr Phe Ile Val Asn Leu Ser Leu Ala Asp Val Leu Val Thr
                85                  90                  95

Ala Ile Cys Leu Pro Ala Ser Leu Leu Val Asp Ile Thr Glu Ser Trp
            100                 105                 110

Leu Phe Gly His Ala Leu Cys Lys
        115                 120

<210>   16
<211>   13
<212>   PRT
<213>   Homo sapiens

<400>   16
Met Asn Leu Pro Ser Thr Lys Val Ser Trp Ala Ala Val
1               5                   10

<210>   17
<211>   12
<212>   PRT
<213>   Influenza A virus

<400>   17
Tyr Asp Lys Glu Glu Ile Arg Arg Ile Trp Arg Gln
1               5                   10

<210>   18
<211>   15
<212>   PRT
<213>   Influenza A virus

<400>   18
Leu Ile Leu Tyr Asp Lys Glu Glu Ile Arg Arg Ile Trp Arg Gln
1               5                   10                  15

<210>   19
<211>   15
<212>   PRT
<213>   Influenza A virus

<400>   19
Leu Ile Leu Tyr Asp Lys Glu Glu Met Arg Arg Ile Trp Arg Gln
1               5                   10                  15

<210>   20
<211>   15
<212>   PRT
<213>   Influenza A virus

<400>   20
Val Gly Lys Met Ile Gly Gly Ile Gly Arg Phe Tyr Ile Gln Met
1               5                   10                  15

<210>   21
<211>   15
<212>   PRT

<213>   Influenza A virus

<400>   21
Ser Gly Ala Ala Gly Ala Ala Val Lys Gly Val Gly Thr Met Val
1               5                   10                  15

<210>   22
<211>   15
<212>   PRT
<213>   Influenza A virus

<400>   22
Glu Lys Ala Thr Asn Pro Ile Val Pro Ser Phe Asp Met Ser Asn
1               5                   10                  15

<210>   23
<211>   15
<212>   PRT
<213>   Influenza A virus

<400>   23
Ile Asp Pro Phe Lys Leu Leu Gln Asn Ser Gln Val Val Ser Leu
1               5                   10                  15

<210>   24
<211>   15
<212>   PRT
<213>   Influenza A virus

<400>   24
Leu Ile Leu Tyr Asp Lys Glu Glu Arg Arg Arg Arg Trp Arg Gln
1               5                   10                  15

<210>   25
<211>   15
<212>   PRT
<213>   Homo sapiens

<400>   25
Met Asn Leu Pro Ser Thr Lys Val Ser Trp Ala Ala Val Thr Leu
1               5                   10                  15

<210>   26
<211>   15
<212>   PRT
<213>   Homo sapiens

<400>   26
Met Asn Leu Pro Ser Ile Lys Val Ser Trp Ala Ala Val Thr Leu
1               5                   10                  15

<210>   27
<211>   15
<212>   PRT
<213>   Homo sapiens

<400>   27
Met Asn Leu Pro Ser Met Lys Val Ser Trp Ala Ala Val Thr Leu
1               5                   10                  15

<210>   28
<211>   15
<212>   PRT

<213>   Influenza A virus

<400>   28
Leu Thr Val Ala Ala Trp Ser Val Lys Thr Ser Pro Leu Asn Met
1               5                   10                  15

<210>   29
<211>   15
<212>   PRT
<213>   Homo sapiens

<400>   29
Gly Ala Gly Asn His Ala Ala Gly Ile Leu Thr Leu Gly Lys Arg
1               5                   10                  15

<210>   30
<211>   15
<212>   PRT
<213>   Homo sapiens

<400>   30
Ala Ser Gly Asn His Ala Ala Gly Ile Leu Thr Met Gly Arg Arg
1               5                   10                  15

<210>   31
<211>   15
<212>   PRT
<213>   Influenza A virus

<400>   31
Ala Met Glu Arg Asn Ala Gly Ser Gly Ile Ile Ile Ser Asp Thr
1               5                   10                  15

<210>   32
<211>   15
<212>   PRT
<213>   Influenza A virus

<400>   32
Ala Leu Asn Arg Gly Ser Gly Ser Gly Ile Ile Thr Ser Asp Ala
1               5                   10                  15

<210>   33
<211>   15
<212>   PRT
<213>   Influenza A virus

<400>   33
Ala Leu Ser Arg Gly Phe Gly Ser Gly Ile Ile Thr Ser Asn Ala
1               5                   10                  15

## Description

This application claims the benefit of United States provisional applications 61/822,228 (filed May 10, 2013), 61/859,113 (filed July 26, 2013), and 61/862,807 (filed August 6, 2013) and European patent applications 13181429.5 (filed August 23, 2013) and 14158999.4 (filed March 11, 2014), the complete contents of each of which are hereby incorporated herein by reference for all purposes.

### TECHNICAL FIELD

This invention is in the field of providing influenza vaccines and methods for producing influenza vaccines which further reduce the risk of narcolepsy in the vaccine recipient.

### BACKGROUND ART

After the H1N1 swine flu vaccination campaign in 2009, an epidemiological association of narcolepsy with the use of an AS03-adjuvanted H1N1 vaccine (Pandemrix™, GSK Biologicals) was detected in children and adolescents 4-19 years of age by Finland's National Institute of Health and Welfare [1-3]. The incidence of narcolepsy was 9/100,000 in the vaccinated population as compared to 0.7 /100,000 in the unvaccinated individuals, the rate ratio being 12.7 with an onset approximately two months after vaccination [4]. Subsequently, there were similar reports with the AS03-adjuvanted H1N1 vaccine in France, Ireland, Norway, Sweden and Great Britain. Detailed follow-up studies in Finland have further strengthened the initial association from 2011 [5]. A statistical association between Pandemrix™ and narcolepsy has therefore clearly been demonstrated. Interestingly, no increased occurrence of narcolepsy was observed in patients receiving the Focetria™ vaccine which protects against the same H1N1 influenza strain but which comprises a different oil-in-water emulsion adjuvant.

Although the risk of developing narcolepsy in response to an influenza vaccine is very small, it is nevertheless desirable to reduce the risk of narcolepsy further.

### SUMMARY OF THE INVENTION

The invention thus provides methods and vaccines which reduce the likelihood further that a patient will develop narcolepsy in response to an influenza vaccine.

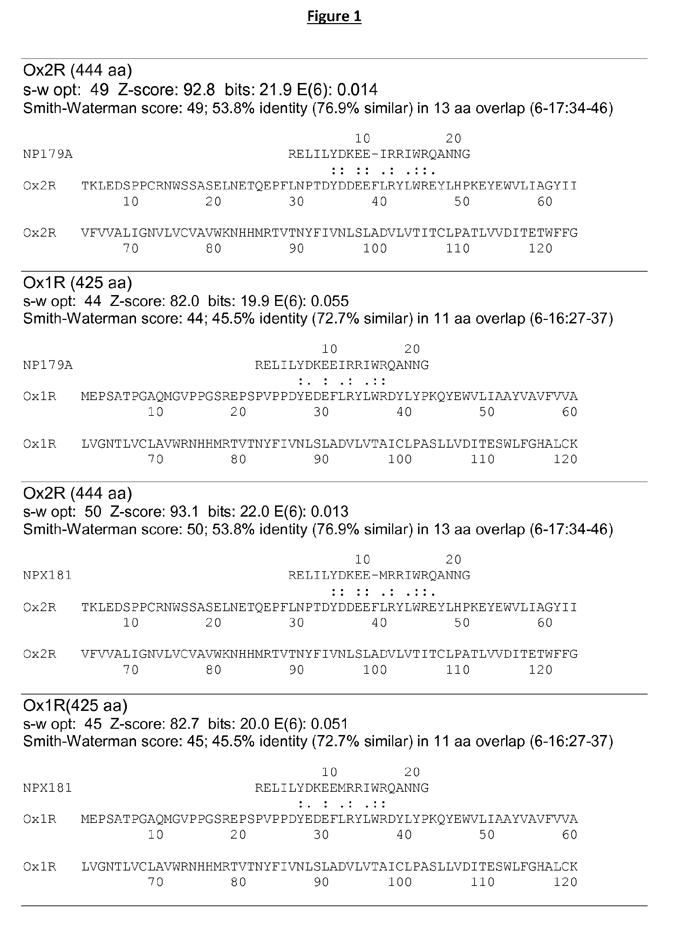

The fact that Pandemrix™ and Focetria™ have different oil-in-water emulsion adjuvants led to the widespread assumption that the adjuvant might be implicated in the development of narcolepsy. The inventors have surprisingly discovered, however, that the causative factor is likely instead to be the different nucleoproteins in the two vaccines, which in Pandemrix™ can mimic the orexin receptor (see Figure 1). Narcolepsy has been associated with the loss of neurons that produce orexin (also known as hypocretin). Moreover, all the cases of Pandemrix™-induced narcolepsy were found in patients which carry the HLA DQB1*0602 haplotype (an MHC class II receptor beta-chain protein: UniProtKB/Swiss-Prot: P01920.2), and such individuals are particularly susceptible to developing narcolepsy (this haplotype being seen in more than 85% of patients diagnosed with narcolepsy with cataplexy and in 50% of patients with less severe forms of narcolepsy). Without wishing to be bound by theory, the inventors thus propose that the observed increase in narcolepsy could be caused by binding of MHC class II receptors comprising HLA DQB 1*0602 to the influenza virus's NP protein, or a fragment thereof, which could trigger an autoimmune reaction that results in disrupting the transmission of the hypocretin signal and/or by the loss of cells that express orexin receptors, thus leading to narcolepsy. In support of this, the data provided herein show that certain peptides derived from the nucleoprotein variant found in the Pandemrix™ vaccine show stable binding to MHC class II receptors comprising HLA DQB1*0602 whereas corresponding peptides from another nucleoprotein do not. This contrasts with much less stable binding of these same peptides to another HLA subtype that has not been associated with narcolepsy.

The invention thus provides methods and vaccines which further reduce the likelihood that a vaccine comprises a nucleoprotein which might be involved in the development of narcolepsy.

One approach would be to select influenza A nucleoproteins with reduced or no binding to MHC class II receptors comprising HLA DQB1*0602. The resulting vaccine composition would therefore lack nucleoproteins that show significant binding to MHC class II receptors comprising HLA DQB1*0602, thereby reducing the risk of triggering the development of narcolepsy in a susceptible individual.

Accordingly, in a first aspect, the present invention provides an influenza vaccine composition comprising influenza A virus nucleoprotein wherein a fragment of said nucleoprotein equivalent to amino acids 106 to 118 of SEQ ID NO: 2 binds to an MHC class II receptor comprising HLA DQB1*0602 with a lower affinity than a peptide having the amino acid sequence shown in SEQ ID NO:1.Preferably if all influenza A nucleoprotein in the composition comprises, or is derived from, the sequence shown in SEQ ID NO: 12, then the vaccine composition is not based on strain A/California/7/2009 (H1N1)-derived strain NYMC X-181. In a particular embodiment the fragment of said nucleoprotein is equivalent to amino acids 106 to 126 of SEQ ID NO: 2.

An example of a known vaccine composition where all of the (influenza A) nucleoprotein in the composition comprises, or is derived from, the sequence shown in SEQ ID NO: 12 is Focetria™, which is therefore specifically excluded.

In a related aspect, the present invention provides an influenza vaccine composition comprising one or more influenza A virus nucleoproteins wherein none of said nucleoprotein(s) comprise(s) a fragment equivalent to amino acids 106 to 126 of SEQ ID NO: 2 which binds to an MHC class II receptor comprising HLA DQB1*0602 with an equal or higher affinity than a peptide having the amino acid sequence shown in SEQ ID NO:1. Preferably if all influenza A nucleoprotein in the composition comprises, or is derived from, the sequence shown in SEQ ID NO: 12, then the vaccine composition is not based on strain A/California/7/2009 (H1N1)-derived strain NYMC X-181. In a specific embodiment, none of said nucleoprotein(s) comprise(s) a fragment equivalent to amino acids 106 to 126 of SEQ ID NO: 2 which binds to an MHC class II receptor comprising HLA DQB1*0602 with an affinity of more than half, a third or a quarter of the binding affinity that a peptide having the amino acid sequence shown in SEQ ID NO:1 has for an MHC class II receptor comprising HLA DQB1*0602.

In a further related aspect, the present invention also provides an influenza vaccine composition comprising influenza virus A nucleoprotein wherein a fragment of said nucleoprotein equivalent to amino acids 106 to 126 of SEQ ID NO: 2 binds to an MHC class II receptor comprising HLA DQB1*0602 with a lower affinity than a peptide having the amino acid sequence shown in SEQ ID NO: 1. Preferably if said nucleoprotein in the composition comprises, or is derived from, the amino acid sequence shown in SEQ ID NO: 12 then the vaccine composition is not based on strain A/California/7/2009 (H1N1)-derived strain NYMC X-181.In a particular embodiment, not all of the influenza A nucleoprotein in the composition comprises, or is derived from, the sequence shown in SEQ ID NO: 12.

In a related embodiment, not all of the influenza A nucleoprotein in the composition comprises the sequence shown in SEQ ID NO: 3.

The term "derived from" mentioned in the various aspects and embodiments above means that shorter fragments may be present as a result of, for example, protein degradation.

The present inventors have identified that a likely key sequence involved in binding of influenza virus A nucleoprotein to HLA DQB1*0602 is an isoleucine residue at a position corresponding to amino acid 116 of the nucleoprotein sequence shown in SEQ ID NO: 2. It is therefore desirable to use influenza A nucleoproteins during vaccine production that lack an isoleucine at this position.

Accordingly, the present invention also provides an influenza vaccine composition comprising influenza A virus nucleoprotein wherein said nucleoprotein does not have an isoleucine residue at a position corresponding to amino acid 116 of the nucleoprotein sequence shown in SEQ ID NO: 2, with the proviso that if all nucleoprotein in the composition comprises the sequence shown in SEQ ID NO: 12, then the vaccine composition is not based on strain A/California/7/2009 (H1N1)-derived strain NYMC X-181.

In a related embodiment, the present invention relates to an influenza vaccine composition comprising influenza virus A nucleoprotein wherein said nucleoprotein does not have an isoleucine or a methionine residue at a position corresponding to amino acid 116 of the nucleoprotein sequence shown in SEQ ID NO: 2.

In another embodiment, the present invention provides an influenza vaccine composition comprising influenza virus A nucleoprotein wherein said nucleoprotein does not comprise the sequence shown in SEQ ID NO: 2 or SEQ ID NO: 12. Preferably the nucleoprotein also does not comprise the sequence shown in SEQ ID NO: 13. In a related embodiment, said nucleoprotein does not comprise the sequence shown in SEQ ID NO: 3. In another related embodiment said nucleoprotein does not comprise the sequence shown as SEQ ID NO: 10.

It should be noted in all of the above embodiments that reference to influenza virus A nucleoprotein means substantially all, or all, influenza virus A nucleoprotein present in the composition, regardless of whether the nucleoprotein is from a single or multiple sources. "Substantially all" typically means that the nucleoprotein(s) from the influenza A strain(s) on which the composition is based should meet the requirements set out herein but there may be small amounts of nucleoprotein from other sources. Thus "substantially all" typically means at least 99% of the influenza virus A nucleoprotein present in the composition.

Thus, for example, other ways to express the first aspect of the invention:
(i) an influenza vaccine composition comprising influenza virus A nucleoprotein wherein, for all influenza virus A nucleoprotein present in the composition, a fragment of said nucleoprotein equivalent to amino acids 106 to 126 of SEQ ID NO: 2 binds to a MHC class II receptor comprising HLA DQB1*0602 with a lower affinity than a peptide having the amino acid sequence shown in SEQ ID NO: 1. Preferably if all influenza virus A nucleoprotein in the composition comprises, or is derived from the amino acid sequence shown in SEQ ID NO: 12 then the vaccine composition is not based on strain A/California/7/2009 (H1N1)-derived strain NYMC X-181.
(ii) An influenza vaccine composition comprising influenza virus A nucleoprotein from a single or multiple source(s) wherein a fragment(s) of all said nucleoprotein(s) equivalent to amino acids 106 to 126 of SEQ ID NO: 2 bind(s) to a MHC class II receptor comprising HLA DQB1*0602 with a lower affinity than a peptide having the amino acid sequence shown in SEQ ID NO: 1. Preferably if all influenza virus A nucleoprotein in the composition comprises, or is derived from, the amino acid sequence shown in SEQ ID NO: 12 then the vaccine composition is not based on strain A/California/7/2009 (H1N1)-derived strain NYMC X-181.(iii) An influenza vaccine composition comprising influenza virus A nucleoprotein from a single or multiple source(s), wherein said nucleoprotein(s) include(s) fragment(s) of said nucleoprotein equivalent to amino acids 106 to 126 of SEQ ID NO: 2, and wherein said fragment(s) of all influenza virus A nucleoprotein present in the composition bind(s) to an MHC class II receptor comprising HLA DQB1*0602 with a lower affinity than a peptide having the amino acid sequence shown in SEQ ID NO: 1. Preferably if all nucleoprotein in the composition comprises the amino acid sequence shown in SEQ ID NO: 12, then the vaccine composition is not based on strain A/California/7/2009 (H1N1)-derived strain NYMC X-181.

Based on the findings presented herein, a person skilled in the art will be able to take either an existing or newly identified nucleoprotein sequence and make modifications so that the resulting nucleoprotein has reduced, or no, binding to an MHC class II receptor comprising HLA DQB1*0602 as compared with the unmodified nucleoprotein.

Accordingly, the present invention also provides an influenza vaccine composition comprising influenza virus A nucleoprotein wherein the nucleoprotein has been modified to reduce or abolish its binding to an MHC class II receptor comprising HLA DQB1*0602 as compared with the unmodified nucleoprotein.

In a preferred embodiment, the nucleoprotein has been modified to change or delete an isoleucine residue in the position corresponding to amino acid 116 of SEQ ID NO: 2. In an alternative embodiment, the nucleoprotein has been modified to change or delete one or more amino acids such that (a) there is no aliphatic amino acid in the position corresponding to amino acid 108 of SEQ ID NO: 2; and/or (b) there is no aliphatic amino acid in the position corresponding to amino acid 110 of SEQ ID NO: 2; and/or (c) there is no hydrophobic amino acid in the position corresponding to amino acid 111 of SEQ ID NO: 2. These two embodiments may also be combined. Typically, therefore, the resulting nucleoprotein will have a different amino acid sequence from wild type nucleoprotein sequences.

Another approach to avoiding or reducing the risk of narcolepsy is to reduce the amount of nucleoprotein present in the final vaccine composition. Split virion and whole vaccines in particular may contain considerable amounts of residual nucleoprotein.

Accordingly, in a second aspect, the present invention provides an influenza vaccine composition comprising influenza virus A nucleoprotein wherein (i) the composition is a split virion vaccine and the amount of influenza virus A nucleoprotein present is less than 3µg nucleoprotein per 10 µg of hemagglutinin or (ii) the composition is a subunit vaccine and the amount of nucleoprotein present is less than 0.5 µg nucleoprotein per 10 µg of hemagglutinin. Further guidance as to the desired levels of nucleoprotein is given further below.

The first and second aspects of the invention can be combined. Thus in a particular embodiment where a mixture of influenza A nucleoproteins are present (multivalent vaccine), it may only be necessary to reduce the amount of the nucleoprotein to below the level specified herein with respect to that nucleoprotein which is to be avoided as per the first aspect of the invention. Other nucleoprotein, such as that of strain X-181, may be included at, for example, the usual levels for any particular type of vaccine. Thus, in this embodiment, more stringent purification measures need only be applied during the manufacture of antigen for some strain(s) and not others, depending on the sequence/binding characteristics of the particular nucleoprotein. Thus the invention may be applied to a monovalent bulk, which may then be combined with other monovalent bulk(s) which need not been subjected to any special measures, to give a multivalent vaccine with different nucleoproteins.

In a particular embodiment of the first and second aspects of the invention the vaccine composition further comprises an adjuvant, such as an oil-in-water emulsion. Since the increased incidence of narcolepsy was seen in GSK's adjuvanted Pandemrix™ vaccine, this effect may be increased by the presence of an adjuvant and therefore it may be particularly important to apply the teachings of the present invention to adjuvanted vaccines, both seasonal or pandemic. The adjuvant, such as the oil-in-water emulsion, may further comprise tocopherol, as is the case in the AS03 adjuvant used in GSK's adjuvanted Pandemrix™ vaccine. In a particular embodiment, the oil-in-water emulsion MF59 is excluded as an adjuvant.

Process changes may also have an effect on the potentially deleterious effects of certain nucleoproteins. For example, the type of detergent used may have an impact. Accordingly in one embodiment of the first and second aspects of the invention, the vaccine composition further comprises Triton (*e.g.* Triton X-100 or t-octylphenoxypolyethoxyethanol) or Tween (*e.g.* Tween-80 or polysorbate 80), or a combination thereof. Such vaccine compositions may be adjuvanted or unadjuvanted as described in the previous paragraph.

The vaccine compositions of the first and second aspects of the invention may be a vaccine against one or more pandemic influenza strains and/or against one or more seasonal influenza strains. In one embodiment the vaccine composition is a monovalent composition e.g. contains one pandemic influenza strain only. In a related embodiment the vaccine composition comprises influenza A strains only.

In one embodiment of the first and second aspects of the invention, the vaccine is a tetravalent vaccine.

The vaccine composition of the first and second aspects of the invention may, for example, be a whole virus vaccine, a split virion vaccine or a subunit vaccine. In one embodiment, the vaccine composition is a split virion vaccine.

In another embodiment, where the vaccine compositions of the invention are against pandemic viruses, the vaccine compositions are not obtained from reassortant viruses NYMC X-157, X163, X-163A, X-163B, X-173, X-173A, X-173B, X-173C, X-177, X-177A, X-177B, X-179, X-179A, X-181 or X-181B.

In another embodiment, where the vaccine compositions of the invention are against seasonal viruses, the vaccine compositions are not obtained from reassortant viruses NYMC X-157, X163, X-163A, X-163B, X-173, X-173A, X-173B, X-173C, X-177, X-177A, X-177B, X-179, X-179A, X-181 or X-181B.

In one embodiment, the vaccine is against a H1, H2, H3, H4, H5, H6, H7, H8 or H9 strain, such as a pandemic H1, H3, H5, H6, H7 or H9 strain. Specific examples are H1N1, H5N1, H5N3, H7N9, H9N2, H5N8, H5N9, H7N4, H7N7, H7N3 and H7N1.

Additional specific embodiments are disclosed below:
No significant association has been detected between narcolepsy and immunization with MF59-adjuvanted flu vaccine. Therefore the NP protein might be a particular risk if oil-in-water adjuvants with additional immunostimulating agents are used. Such additional immunostimulating agents could *e.g.* be tocopherol/tocopherol derivatives (AS03), or a TLR agonist, like e.g. the synthetic TLR4 agonist Glucopyranosyl Lipid A (GLA; see WO2009/143457). Therefore, flu vaccines adjuvanted with said oil-in-water adjuvants should preferably be free of influenza A NP protein. Therefore one embodiment of the invention is:
   (a) an inactivated, adjuvanted influenza vaccine which does not contain influenza A NP protein, or contains influenza A NP protein with less than 15%, 12%, 10%, 8%, 7%, 6%, 5%, 4%, 3%, 2%, or 1% by mass of the total influenza virus protein in the vaccine. In a preferred embodiment the adjuvant is an oil-in-water emulsion which contains an additional immunostimulating agent, like a tocopherol, a tocopherol derivative, MPL, GLA or another TLR agonist. In an alternative embodiment, the vaccine is Alum adjuvanted and contains an immunostimulating agent like an adsorbed TLR agonist. In a preferred embodiment, the vaccine is a subunit or a split vaccine.

'Additional immunostimulating agent' in the context of an oil-in-water emulsion means an immunostimulating agent included in addition to the base oil and the detergent(s) which form the emulsion. The additional immunostimulating agent is added to increase the adjuvant effect of the emulsion. For the purpose of this patent, MF59 which consists of squalene, Span and Tween is understood not to contain additional immunostimulating agents. The tocopherol contained in AS03 is understood to be an additional immunostimulating agent. For the purpose of this patent surfactants typically used to form and/or stabilize the emulsion are not regarded as 'additional immunostimulating agent', even if these detergents have a certain intrinsic adjuvant activity. Accordingly, the following detergents are not to understood to be additional immunostimulating agent: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. On the other hand, TLR agonist counts as additional immunostimulating agents for the purpose of this patent, even if they are added in the chemical form of an oil or a surfactant. In particular the TLR agonists described below in the adjuvant section are understood as additional immunostimulating agents.

Alternatively, if the presence of influenza A NP protein cannot completely be avoided in vaccines adjuvanted with oil-in-water emulsions or Alum and additional immunostimulators, a NP protein should be used which is different from the NP protein contained in Pandemrix™. Therefore one embodiment of the invention is:
(b) An adjuvanted influenza vaccine which contains NP protein from an influenza A virus, characterized in that:
   (i) the NP protein does not have an isoleucine residue at a position corresponding to amino acid 116 of the nucleoprotein sequence shown in SEQ ID NO: 2, and/or
   (ii) a fragment of said nucleoprotein equivalent to amino acids 106 to 118 (typically amino acids 106 to 126) of SEQ ID NO: 2 binds to an MHC class II receptor comprising HLA DQB1*0602 with a lower affinity than a peptide having the amino acid sequence shown in SEQ ID NO: 1,
whereby the adjuvant is an oil-in-water emulsion or Alum and an additional immunostimulating agent.

In a preferred embodiment, the adjuvant is an oil-in-water emulsion and the additional immunostimulating agent is a tocopherol, a tocopherol derivative, GLA, MPL or another TLR agonist. In an alternative embodiment the adjuvant is Alum (such as aluminium hydroxide or aluminium phosphate) and contains a TLR agonist.

If the presence of the NP protein with the sequence SEQ ID NO: 2 as used in Pandemrix™ cannot be avoided (e.g. because alternative backbones are not available), the vaccine should only be adjuvanted with an adjuvant that does not contain an additional immunostimulating agent. This applies in particular to (i) split vaccines (which contain a relatively high amount of NP), (ii) tetravalent or higher-valent vaccine (as the amount of NP adds up), (iii) vaccine to be given to the pediatric population and/or the adolescent population (as most of the narcolepsy cases have been detected in these populations), (iv) vaccine to be given to subjects with a genetic predisposition to develop an autoimmune disease in connection with flu vaccination (like subject with HLA DQB1*0602; as narcolepsy has only occurred in these subjects), and (v) to antigens which contain Triton (as narcolepsy has mainly appeared in response to Triton containing antigens). Therefore, one embodiment of the present invention is as follows:
(c) An adjuvanted split influenza vaccine wherein the vaccines contains antigens from at least 4 different influenza viruses and NP protein from at least one influenza A virus with the SEQ ID NO: 2, characterized in that the adjuvant is an oil-in-water emulsion adjuvant which does not contain an additional immunostimulating agent, and whereby the composition contains Triton.

In a preferred embodiment, this vaccines is for use in the pediatric population (0-36 months) and/or the adolescent population (4-19 years) and/or in subjects with a genetic predisposition to develop an autoimmune disease in connection with flu vaccination, preferably subject with HLA DQB1*0602.

An alternative embodiment is:
(d) A method for treatment of a child of 0-72 months, and/or of an adolescent (4-19 years) and/or a subject with a genetic predisposition to develop an autoimmune disease in connection with flu vaccination, preferably a subject with HLA DQB1*0602with HLA DQB whereby the child and/or adolescent and/or subject is vaccinated with an adjuvanted split influenza vaccine wherein the vaccines contains antigens from at least 4 different influenza viruses and NP protein from at least one influenza A virus with the SEQ ID NO: 2, characterized in that the adjuvant is an oil-in-water emulsion adjuvant which does not contain an additional immunostimulating agent, and whereby the composition contains Triton. Further embodiments are:
(e) An inactivated influenza vaccine which contains (i) the AS03 adjuvant, and (ii) an antigen component which contains Triton X-100 and Tween 80, but which either (a) is free from nucleoprotein, or (b) contains nucleoprotein, but the amount of nucleoprotein is less than 1% of the total mass of influenza virus protein in the vaccine.
(f) An inactivated split influenza vaccine which contains (i) the AS03 adjuvant, and (ii) an antigen component which contains Triton X-100 and Tween 80, and which contains nucleoprotein, but which does not contain nucleoprotein or a nucleoprotein fragment which can bind to an MHC class II receptor comprising HLA DQB1*0602. Preferably the vaccine is against an influenza strain associated with a pandemic and the influenza strain is selected from H1N1, H7N9, H9N2, H5N1, H5N3, H5N8, H5N9, H7N4, H7N7, H7N3, H2N2, H10N7, and H7N1.
(g) An inactivated split influenza vaccine against an influenza strain associated with a pandemic which contains (i) the AS03 adjuvant, and (ii) an antigen component which contains Triton X-100 and Tween 80, and which contains nucleoprotein, but which does not contain nucleoprotein or a nucleoprotein fragment which contains isoleucine in the position corresponding to amino acid 116 of SEQ ID NO: 2. Preferably the vaccine is against an influenza strain associated with a pandemic and the influenza strain is selected from H1N1, H5N1, H5N3, H7N9, H9N2, H5N8, H5N9, H7N4, H7N7, H7N3, H2N2, H10N7, and H7N1.
(h) An inactivated split influenza vaccine against an influenza strain associated with a pandemic which contains (i) the AS03 adjuvant, and (ii) an antigen component which contains Triton X-100 and Tween 80, and which contains nucleoprotein, but which does not contain nucleoprotein or a nucleoprotein fragment which contains the sequence LXLYXXXIXXXXXX (SEQ ID NO: 9), wherein X is any amino acid. Preferably the vaccine is against an influenza strain associated with a pandemic and the influenza strain is selected from H1N1, H5N1, H5N3, H7N9, H9N2, H5N8, H5N9, H7N4, H7N7, H7N3, H2N2, H10N7, and H7N1.

In one embodiment, the antigen component of the non-adjuvanted vaccines described above may be used for formulation with an oil-in-water adjuvant, in particular with AS03.
(i) A monovalent influenza vaccine which contains NP protein from an influenza A virus, characterized in that:
   (i) the NP protein does not have an isoleucine residue at a position corresponding to amino acid 116 of the nucleoprotein sequence shown in SEQ ID NO: 2, and/or
   (ii) a fragment of said nucleoprotein equivalent to amino acids 106 to 118 (or 106 to 126) of SEQ ID NO: 2 binds to an MHC class II receptor comprising HLA DQB 1*0602 with a lower affinity than a peptide having the amino acid sequence shown in SEQ ID NO: 1,
   whereby the vaccine contains less than 7.5 micrograms of HA per dose, and optionally an adjuvant.
(j) An influenza vaccine containing antigens from at least 4 different influenza viruses and NP protein from at least one influenza A virus, characterized in that:
   a. None of the NP protein has an isoleucine residue at a position corresponding to amino acid 116 of the nucleoprotein sequence shown in SEQ ID NO: 2, and/or
   b. A fragment of said at least one nucleoprotein equivalent to amino acids 106 to 118 of SEQ ID NO: 2 binds to an MHC class II receptor comprising HLA DQB 1*0602 with a lower affinity than a peptide having the amino acid sequence shown in SEQ ID NO: 1,
   whereby the vaccine contains optionally an adjuvant.
(k) An adjuvanted inactivated split influenza vaccine which includes an influenza A virus nucleoprotein, wherein the vaccine is free from (i) influenza A virus nucleoprotein having an isoleucine residue at a position corresponding to amino acid 116 of the nucleoprotein sequence shown in SEQ ID NO: 2 and (ii) influenza A virus nucleoprotein from A/California/7/2009 (H1N1)-derived strain NYMC X-181. As explained elsewhere herein, this vaccine can be free from the nucleoprotein from any of strains NYMC X-157, X163, X-163A, X-163B, X-173, X-173A, X-173B, X-173C, X-177, X-177A, X-177B, X-179, X-179A, X-181 or X-181B. The vaccine can include an oil-in-water emulsion adjuvant, such as MF59 or AS03. The vaccine can be monovalent or multivalent (e.g. 3-valent or 4-valent).
(1) An adjuvanted inactivated split influenza vaccine which includes an influenza A virus nucleoprotein, wherein the vaccine is free from (i) influenza A virus nucleoprotein having an isoleucine residue at a position corresponding to amino acid 116 of the nucleoprotein sequence shown in SEQ ID NO: 2 and (ii) hemagglutinin from any influenza A virus strain which has been recommended for inclusion in influenza vaccines by the World Health Organization or by the Vaccines and Related Biological Products Advisory Committee prior to 1st October 2013. The vaccine may also be free from (iii) hemagglutinin from any influenza B virus strain which has been recommended for inclusion in influenza vaccines by the World Health Organization or by the Vaccines and Related Biological Products Advisory Committee prior to 1st October 2013. The WHO's strain recommendations are available on the internet [6], and the strains for use in the 2014 southern hemisphere influenza season were announced on 26th September 2013. Recommendations from the FDA's VRBPAC are similarly available online [7], and the strains for use in the 2013/14 influenza season were announced on 27th February 2013. Thus the strains defined by feature (ii) can readily be determined. The vaccine can include an oil-in-water emulsion adjuvant, such as MF59 or AS03. The vaccine can be monovalent but is preferably multivalent (*e.g*. 3-valent or 4-valent). If the vaccine is 4-valent, it ideally includes hemagglutinin from two A and two B strains, where the two A strains have different hemagglutinin subtypes (*e.g*. from H1 and H3, such as an H1N1 strain and an H3N2 strain) and the two B strains have different lineages (*i.e.* a B/Victoria/2/87-like strain and a B/Yamagata/16/88-like strain). See below.

As discussed above, it is desirable to test existing nucleoproteins and in particular new potential nucleoproteins used in influenza vaccine manufacture for potential binding to MHC class II receptor heterodimer where the beta-subunit is HLA DQB 1*0602.

Accordingly, the invention provides a method of testing an influenza A virus for suitability for vaccine production, comprising a step of determining whether the influenza virus's nucleoprotein, or a fragment thereof, or the nucleoprotein encoded by the influenza virus's genome segment, or a fragment thereof can bind to HLA DQB1*0602 with lower affinity under the same conditions compared to a nucleoprotein from H1N1 strain X-179A; wherein the influenza virus is suitable for vaccine production if its nucleoprotein protein can bind to HLA DQB1*0602 with lower affinity under the same conditions compared to nucleoprotein from X-179A. It will be understood by a person skilled in the art that throughout the specification, reference to 'binding to HLA DQB1*0602' means binding to an MHC class II receptor heterodimer where the beta-subunit is HLA DQB 1*0602.

Also provided is a method of testing an influenza A virus for suitability for vaccine production, comprising the steps of (a) determining the sequence of the influenza virus's nucleoprotein, or the influenza virus's genome segment encoding the nucleoprotein; and (b) determining whether the nucleoprotein has a sequence which allows it to bind to HLA DQB 1*0602 with lower affinity under the same conditions compared to a nucleoprotein from strain X-179A; wherein the influenza virus is suitable for vaccine production if its nucleoprotein can bind to HLA DQB 1*0602 with lower affinity under the same conditions compared to nucleoprotein from strain X-179A.

As mentioned above, the binding of the virus's NP protein to an MHC class II receptor including HLA DQB 1*0602 can be involved in disease development and it is therefore desirable in vaccines to choose an influenza A virus whose NP protein has a lower binding affinity for HLA DQB 1*0602 compared to strain X-179A (which was used for PandemriX™). This NP protein has nascent amino acid sequence SEQ ID NO: 2, and analysis below indicates that an important part of this sequence for binding HLA DQB1*0602 is RELILYDKEEIRRIWRQANNG (SEQ ID NO: 1).

The NP protein of the influenza virus used in Focetria™, which did not trigger narcolepsy, differs from the NP protein of Pandemrix™ in that it does not contain isoleucine in the position corresponding to amino acid 116 of SEQ ID NO: 2 (see Figure 1) and it is therefore desirable to avoid a NP protein which has isoleucine in this position. Interestingly, our analysis of the database sequences of thousands of known NP proteins has shown that this region shows a high degree of conservation and that the majority of known NP proteins, especially those found in reassortant viruses used for vaccine manufacture, have an isoleucine at this position or its equivalent.

The invention also provides a method of testing an influenza A virus for suitability for vaccine production, comprising the steps of (a) determining the sequence of the influenza A virus's nucleoprotein or the influenza virus's genome segment encoding the nucleoprotein; and (b) determining the nucleoprotein's amino acid at the position corresponding to amino acid 116 of SEQ ID NO: 2; wherein the influenza virus is suitable for vaccine production if its NP protein does not contain isoleucine in the position corresponding to amino acid 116 of SEQ ID NO: 2.

Thus, the invention also provides a seed virus which is suitable for the preparation of vaccine composition of the invention. In one embodiment, the seed virus of the invention comprises influenza A virus nucleoprotein wherein a fragment of said nucleoprotein equivalent to amino acids 106 to 118 of SEQ ID NO: 2 binds to an MHC class II receptor comprising HLA DQB1*0602 with a lower affinity than a peptide having the amino acid sequence shown in SEQ ID NO:1. Preferably if the influenza A nucleoprotein in the seed virus comprises the sequence shown in SEQ ID NO: 12, then the virus seed is not the strain A/California/7/2009 (H1N1)-derived strain NYMC X-181. In a particular embodiment the fragment of said nucleoprotein is equivalent to amino acids 106 to 126 of SEQ ID NO: 2.

When assessing the suitability of an influenza A virus for vaccine production, it is further desirable to check whether the virus's nucleoprotein comprises one or more of (a) an aliphatic amino acid in the position corresponding to amino acid 108 of SEQ ID NO: 2; and/or (b) a aliphatic amino acid in the position corresponding to amino acid 110 of SEQ ID NO: 2; and/or (c) an hydrophobic amino acid in the position corresponding to amino acid 111 of SEQ ID NO: 2. The core binding motifs of the nucleoprotein for binding to HLA DQB1*0602 are at amino acids 108, 110, 111, 113 and 116 of SEQ ID NO: 2, wherein binding works particularly well when amino acids 108 and 110 are aliphatic amino acids and the amino acid at position 111 is a hydrophobic amino acid. Avoiding such amino acids at these positions thus decreases the likelihood that the NP protein can bind to HLA DQB which further decreases the likelihood that the NP protein will cause narcolepsy. An influenza virus is considered particularly suitable for vaccine production if it does not contain one, two or all of these specific amino acids. The binding pocket at position 111 has been shown to be particularly important for binding and it is therefore preferred that the NP protein does not contain a hydrophobic amino acid at this position. It is particularly preferred that the protein does not have tyrosine in this position because known examples of strong binders have this amino acid [8]. The amino acids at positions 108 and/or 110 are preferably not leucine. The influenza virus may also be considered suitable for vaccine production if it does not comprise the sequence LXLYXXXIXXXXXX (SEQ ID NO: 9), wherein X is any amino acid.

The invention also provides a method of preparing an influenza A virus, comprising the steps of (a) testing the suitability of the influenza A virus for vaccine production by a method of the invention; (b) infecting a culture host with the influenza virus from step (a); and (c) culturing the host from step (b) to produce further virus; and optionally (d) purifying virus obtained in step (c). In these methods, the virus can be used for vaccine production if it is considered suitable for vaccine production, as assessed by a method of the invention.

The invention further provides a method of confirming that a vaccine comprising an influenza A nucleoprotein or a fragment thereof is suitable for administration to a human, comprising a step of determining whether the protein has isoleucine in the position corresponding to amino acid 116 of SEQ ID NO: 2; wherein the vaccine is considered suitable for administration to a human if the nucleoprotein does not contain isoleucine in the position corresponding to amino acid 116 of SEQ ID NO: 2.

The method may further comprise testing whether, as discussed above, the virus's nucleoprotein comprises one or more of (a) an aliphatic amino acid in the position corresponding to amino acid 108 of SEQ ID NO: 2; and/or (b) a aliphatic amino acid in the position corresponding to amino acid 110 of SEQ ID NO: 2; and/or (c) an hydrophobic amino acid in the position corresponding to amino acid 111 of SEQ ID NO: 2. The method may comprise testing whether the virus's nucleoprotein has all of these amino acids.

As discussed above, influenza A viruses whose NP protein (or a fragment thereof) can bind to HLA DQB1*0602, or whose NP protein has isoleucine in the position corresponding to amino acid 116 of SEQ ID NO: 2, have been associated with narcolepsy. It is thus desirable to avoid the presence of such NP proteins or fragments as this would increase the confidence in influenza A vaccines further and would also increase the safety of the vaccine further. Thus, where an influenza A virus which can bind HLA DQB1*0602 and/or which has isoleucine in the position corresponding to amino acid 116 of SEQ ID NO: 2 is used for vaccine production, it is preferred that the resulting vaccine is tested to ensure that the NP protein (or a fragment thereof) is not present in the final vaccine.

Therefore the invention also provides a method of confirming that an influenza vaccine is safe for administration to humans. The method may comprise the steps of (a) preparing an influenza vaccine from an influenza virus whose nucleoprotein can bind to HLA DQB1*0602 (for example, a strain whose NP is SEQ ID NO: 2); and (b) testing the vaccine for the presence of the nucleoprotein; wherein the vaccine is safe for administration to humans if it does not contain the nucleoprotein which can bind to HLA DQB1*0602. Alternatively, it may comprise the steps of (a) preparing an influenza vaccine from an influenza virus whose nucleoprotein has isoleucine in the position corresponding to amino acid 116 of SEQ ID NO: 2; and (b) testing the vaccine for the presence of the nucleoprotein; wherein the vaccine is considered safe for administration to humans if it does not contain the nucleoprotein which can bind to HLA DQB1*0602.

As discussed above, all cases of narcolepsy in response to the Pandemrix™ vaccine occurred in patients who had the HLA DQB1*0602 haplotype and it is therefore desirable to exercise specific caution with this patient group. It is therefore desirable to take the patient's HLA haplotype into account before administering a vaccine.

Thus the invention also provides a method of administering a vaccine to a subject; comprising the steps of: (a) determining whether the subject has a genetic pre-deposition to develop an autoimmune disease in connection with the vaccine, and (b) administering the vaccine if the subject is found to be not at risk. In a preferred embodiment the invention provides a method for administering a vaccine to a subject comprising the steps of (a) determining whether the subject has a certain HLA haplotype; and (b) administering vaccine to the subject if the subject is found to be negative for said haplotype. In a particularly the invention provides a method for administering an influenza vaccine to a subject comprising the steps of (a) determining whether the subject has a HLA DQB1*0602 haplotype, and (b) administering an influenza vaccine to the subject if the subject is negative for HLA DQB1*0602.

The present invention also provides a method of immunizing a subject which method comprises administering a vaccine to the subject wherein the subject is negative for a HLA which poses a risk to develop an autoimmune disease in connection with the vaccine. In preferred embodiment that vaccine is an influenza A vaccine and/or the subject is negative for HLA DQB1*0602.

Alternatively, the present invention provides a vaccine for use in a subject with a HLA haplotype that poses a risk for autoimmune disease in connection with said vaccine, whereby a vaccine is substantially free from a vaccine component which binds to that HLA haplotype. In a preferred embodiment the vaccine is against an influenza A virus and the HLA type DQB and the vaccine does not contain NP protein, or contains an NP protein that does not have an isoleucine residue at a position corresponding to amino acid 116 of the nucleoprotein sequence shown in SEQ ID NO: 2, and/or the NP protein or a fragment of said nucleoprotein equivalent to amino acids 106 to 118 of SEQ ID NO: 2 binds to an MHC class II receptor comprising HLA DQB 1*0602 with a lower affinity than a peptide having the amino acid sequence shown in SEQ ID NO 1. In a preferred embodiment the NP protein or fragment therefrom has been removed. In more preferred embodiment, the influenza vaccine is a not a recombinant vaccine.

The present invention also provides a method of immunizing a subject against influenza which method comprises administering an influenza vaccine to a subject wherein the subject is positive for HLA DQB whereby the vaccine does not contain influenza A NP protein, or contains an influenza A NP protein that does not have an isoleucine residue at a position corresponding to amino acid 116 of the nucleoprotein sequence shown in SEQ ID NO: 2, and/or the NP protein or a fragment of said nucleoprotein equivalent to amino acids 106 to 118 of SEQ ID NO: 2 binds to an MHC class II receptor comprising HLA DQB1*0602 with a lower affinity than a peptide having the amino acid sequence shown in SEQ ID NO 1. In a preferred embodiment, the vaccine is a recombinant vaccine.

The present invention also provides an influenza vaccine containing a NP for use in a subject with HLA DQB1*0602 whereby the NP protein comprises the sequence SEQ ID NO: 2.

The invention also provides an influenza vaccine prepared from an influenza virus whose nucleoprotein, or a fragment thereof, can bind to HLA DQB wherein the vaccine does not contain a nucleoprotein, or a fragment thereof, which can bind to HLA DQB1*0602. In a preferred embodiment, the influenza vaccine is a not a recombinant vaccine.

Further provided is an influenza vaccine prepared from an influenza virus whose nucleoprotein has been tested for ability to bind to HLA DQB1*0602 with equal or higher affinity compared to a nucleoprotein comprising the sequence of SEQ ID NO: 1, wherein the vaccine does not comprise the nucleoprotein. In a preferred embodiment, the influenza vaccine of the invention is a not a recombinant vaccine.

Also provided is an influenza vaccine prepared from an influenza virus whose nucleoprotein has been tested for ability to bind to HLA DQB1*0602 with equal or higher affinity compared to a nucleoprotein from strain X-179A, wherein the vaccine does not comprise the nucleoprotein. In a preferred embodiment, the influenza vaccine of the invention is a not a recombinant vaccine.

Also provided is an influenza vaccine prepared from an influenza virus whose nucleoprotein, or a fragment thereof, has been tested for the presence of isoleucine in the position corresponding to amino acid 116 of SEQ ID NO: 2, wherein the vaccine does not comprise the nucleoprotein or a fragment thereof. In a preferred embodiment, the influenza vaccine of the invention is a not a recombinant vaccine.

The invention also provides a split virion influenza vaccine which comprises a lower amount of nucleoprotein from influenza A virus relative to HA than the Pandemrix™ vaccine. This can be an important safety measure, especially where the nucleoprotein has not been tested as described herein because a lower amount of nucleoprotein makes it less likely that the nucleoprotein will trigger narcolepsy. In a particular embodiment the present invention provides split virion vaccine compositions wherein the amount of nucleoprotein present is less than 3µg nucleoprotein per 10 µg of hemagglutinin, such as less than 3µg NP per 10µg of HA, less than 2.5µg NP per 10µg of HA, less than 2µg NP per 10µg of HA, less than 1.5µg NP per 10µg of HA, less than 1µg NP per 10µg of HA, less than 0.5µg NP per 10µg of HA or less than 0.1µg NP per 10µg of HA.

Methods to determine to amount of protein in a composition are known to the skilled person in the art. However, since NP and NA have virtually the same molecular weight (around 60 kDa), they usually co-migrate in non-reducing gels. Classical SDS gel-electrophoresis might therefore not be an appropriate way to determine the amount of NP [9]. One way to determine the amount of NP in a vaccine bulk might be a 2 dimensional electrophoresis with a subsequent densitometry. Preferred, however, is isotope dilution mass spectrometry using an isotopically labeled synthetic peptide as described in ref. 10. This method uses liquid chromatography-tandem mass spectrometry (LC-MS/MS) using isotope dilution in conjunction with multiple reaction monitoring (MRM). This method quantifies targeted peptides released by proteolytic digestion of the sample as a stoichiometric representative of the analyte protein. A stable isotope-labeled reference peptide is spiked into the sample as an internal standard (IS). Quantification of NP is achieved by comparing the peak area of the isotopically labeled reference peptide with that of the endogenous target peptide.

This method allows simultaneous quantification of multiple proteins, provided labeled peptides are included for each specific target.

Alternatively, label free mass spectrometry (LC/MSE) is used for the quantification, preferably in quadrupole time-of-flight (Q-Tof) mass spectrometers [11]. For this method, alternating scans of low collision energy and elevated collision energy during LC/MS analysis are used to obtain both protein identity and quantity in a single experiment. Quantification is based on the experimental data showing that the average signal intensity measured by LC/MSE of the three most intense tryptic peptides for any given protein is constant at a given concentration, regardless of protein type and size. As the signal intensity is proportional to concentration, the amount of any protein in the mixture can be estimated.

The invention also provides a split virion influenza vaccine wherein the ratio of nucleoprotein to hemagglutinin is less than 1.5 *(e.g.* <1.4, <1.3, <1.2, <1.1, or even <1.0) as assessed by the following assay: proteins in the vaccine are precipitated; the precipitated proteins are collected, reduced, and alkylated using propionamide; the alkylated proteins are digested overnight at 37°C with a mixture of trypsin and LysC (serine endoproteinase from *Lysobacter enzymogenes* which hydrolyses specifically at the carboxyl side of Lys residues); acidification with formic acid; purification of proteolytic fragments using a C18 reversed-phase resin; HPLC-MSMS (high-performance liquid chromatography tandem mass spectrometry) of the purified fragments, with selection of the 15 most intense multiply charged precursor ions for fragmentation; match MS spectral peaks to influenza virus proteins; select the ten most abundant proteins in the MS spectrum; and calculate the ratio of nucleoprotein MS signal to hemagglutinin MS signal within these ten most abundant proteins. As shown below, in current split vaccines the ratio of NP to MS, assessed by this assay, is more than 1.5.

The invention also provides a split virion influenza vaccine wherein the ratio of nucleoprotein to hemagglutinin is less than 0.3 *(e.g.* <0.28, <0.26, <0.25, <0.20, or even <0.10) as assessed by the following assay: proteins in the vaccine are precipitated; the precipitated proteins are collected, reduced, and alkylated using propionamide; the alkylated proteins are digested overnight at 37°C with a mixture of trypsin and LysC (serine endoproteinase from *Lysobacter enzymogenes* which hydrolyses specifically at the carboxyl side of Lys residues); acidification with formic acid; purification of proteolytic fragments using a C18 reversed-phase resin; HPLC-MSMS (high-performance liquid chromatography tandem mass spectrometry) of the purified fragments, with selection of the 15 most intense multiply charged precursor ions for fragmentation; match MS spectral peaks to influenza virus nucleoprotein and hemagglutinin sequences by precise sequence match to strains known to be present in the vaccine; select the ten most abundant proteins in the MS spectrum; and calculate the ratio of nucleoprotein MS signal to hemagglutinin MS signal within these ten most abundant proteins.

The invention also provides a subunit influenza vaccine which comprises reduced levels of influenza A nucleoprotein, noting that nucleoprotein levels should be lower in subunit vaccines than split virion vaccines due to the additional purification steps performed. In particular, the present invention provides a subunit vaccine wherein the amount of nucleoprotein present is less than 0.5µg NP per 10µg of HA, such as less than 0.1µg NP per 10µg of HA. The vaccines of the preceding paragraphs are advantageous because, where a vaccine has been prepared from an influenza virus whose NP protein shares characteristics with the NP protein of an influenza virus that was used for the production of an influenza vaccine that caused narcolepsy, it is desirable to ensure that the vaccine does not comprise the NP protein in concentrations which have been associated with narcolepsy.

Typically, the vaccines described in the previous paragraphs are adjuvanted with an oil-in-water emulsion adjuvant. In such vaccines, like Pandemrix™, it is particularly advantageous to test the vaccine because, as explained below, the adjuvant may lead to a higher risk that the NP peptide can trigger narcolepsy. In a most preferred embodiment, the oil-in-water emulsion adjuvant comprises an additional immunostimulating agents, like a tocopherol, a tocopherol derivative, GLA or a TLR agonist, in particular α-tocopherol,

The influenza vaccines are produced from an influenza virus which has been tested for the characteristics discussed in the preceding paragraphs. This testing step can be performed at any stage during the production process. It can be performed, for example, on the seed virus which is used to start the viral culture from which the vaccine is produced. It can also be performed on a sample taken from the viral culture. The testing can also be performed on an influenza virus which is not used in the production process, for example where the testing is performed using one batch of a viral strain and another batch of the same viral strain is used for the production of the vaccine. The testing does not need to be performed in every production cycle. It also does not need to be performed by the same entity that produces the influenza vaccine. For example, it is possible for one entity to test the virus and to make the test results available to other entities, for example in a database. This option is particularly attractive where the testing is done by obtaining the influenza virus's sequence information as such information can easily be made available in a public database.

Also provided is a method for preparing an influenza A vaccine, comprising steps of: (a) preparing a first pre-vaccine composition; (b) removing or reducing the amount of composition structures which mimic orexin receptor 1 and/or orexin receptor 2 from the first vaccine, to provide a second pre-vaccine; and (c) preparing the vaccine from the second pre-vaccine. Preferably, the amount of structures which mimic orexin receptor 1 and/or orexin receptor 2 are reduced by more than 90%, more preferably by 95% and most preferred by more than 99%. As an example, the removal in step (b) is by chromatography. The invention also provides a method for preparing viral subunits from a virus, wherein the subunits are prepared free from structures which mimic orexin receptor 1 and/or orexin receptor 2. The method might include further steps like formulating the vaccine, optionally mixing the vaccine with an adjuvant, filling, packaging and labeling the vaccine.

The invention further provides a method for preparing a vaccine from an influenza virus whose nucleoprotein sequence comprises the amino acid sequence RELILYDKEEMRRIWRQANNG (SEQ ID NO: 3), comprising a step of removing any nucleoprotein, or fragments thereof, which include said amino acid sequence, to provide the vaccine. In another embodiment, the vaccine of the invention is prepared from an influenza virus whose nucleoprotein sequence comprises the amino acid sequence RELILYDKEEIRRIWRQANNG (SEQ ID NO: 3), comprising a step of removing any nucleoprotein, or fragments thereof, which include said amino acid sequence, to provide the vaccine. The methods might include further steps like formulating the vaccine, optionally mixing the vaccine with an adjuvant, filling, packaging and labeling the vaccine.

The invention also provides an inactivated or live attenuated influenza A vaccine wherein the vaccine does not comprise the nucleoprotein or a fragment thereof which can bind to HLA DQB 1*0602. The invention also provides an inactivated or live attenuated influenza A vaccine which does not contain a nucleoprotein, or a fragment thereof, which mimic orexin receptor 1 and/or orexin receptor 2. Also provided is an influenza A vaccine that comprises a nucleoprotein but where the amount of nucleoprotein is less than 3µg NP per 10µg of HA, less than 2.5µg NP per 10µg of HA, less than 2µg NP per 10µg of HA, less than 1.5µg NP per 10µg of HA, less than 1µg NP per 10µg of HA, less than 0.5µg NP per 10µg of HA or less than 0.1µg NP per 10µg of HA. The invention also provides an inactivated influenza A vaccine does not contain a nucleoprotein, or a fragment thereof. Preferably the vaccines described in this paragraph have been produced starting from an influenza A virus which contained a nucleoprotein or a fragment which can bind to HLA DQB 1*0602. In addition, or alternatively, said vaccines are adjuvanted with an oil-in-water emulsion, in particular those containing an additional immunostimulator.

The invention further provides a method for preparing an influenza A vaccine, comprising steps of removing or reducing the amount of composition structures which mimic orexin receptor 1 and/or orexin receptor 2, or fragments thereof. Preferably, the amount of structures which mimic orexin receptor 1 and/or orexin receptor 2 are reduced by more than 90%, more preferably by 95% and most preferred by more than 99%. The invention further provides a method for preparing an influenza A vaccine, comprising steps of removing or reducing the amount of the nucleoprotein or fragments thereof. Preferably, the amount of nucleoprotein or fragments are reduced by more than 90%, more preferably by 95% and most preferred by more than 99%. Methods for removing a protein from a preparation are well known in the art. As an example, removing or reducing the amount of the nucleoprotein or fragments thereof can be done by chromatography or immunoprecipitation.

As narcolepsy has been detected mostly in the pediatric (0-36 months) and the adolescent (4-19 years) population, in one embodiment all vaccines of the invention as described herein are for use in the pediatric (0-36 months) and the adolescent (4-19 years) population.

When oil-in-water emulsion are used in combination with the antigens described herein, the antigen and the adjuvant component of the vaccine might be premixed or might be in separate containers for mixture by the end-user/health care provider before administration. The antigen and the adjuvant component might be produced in the same or in different production sites. One aspect of the invention is the use of the antigen components for formulation and/or packaging into a kit with an adjuvant component. Another aspect of the invention is the use of the adjuvant component for formulation and/or packaging into a kit with an antigen component.

### DETAILED DESCRIPTION OF THE INVENTION

### The nucleoproteins

The invention provides methods which allow a skilled person to assess the suitability of an influenza A virus for vaccine production based on certain characteristics of the virus's nucleoprotein, such as binding to HLA DQB 1*0602 or checking the sequence for the presence of certain amino acids.

Where the invention is defined in terms of testing (or sequencing, analysing, *etc.)* nucleoprotein, this can involve testing the full-length nucleoprotein, but will usually involve testing a fragment of the nucleoprotein since the nucleoprotein would be expected to be presented bound to MHC class II receptor as a fragment following intracellular processing by antigen presenting cells. These fragments can have a length of less than 200 amino acids, for example less than 100 amino acids, less than 90 amino acids, less than 80 amino acids, less than 70 amino acids, less than 60 amino acids, less than 50 amino acids, less than 40 amino acids, less than 30 amino acids, or less than 20 amino acids. As will be appreciated by a person skilled in the art, fragments should typically be at least around 12 or 13 amino acids in length to bind to an MHC class II receptor, such as at least 18, 19, 20 or 21 amino acids in length. It is preferred that the methods of the invention are practised using fragments with a length of less than 30 amino acids as such fragments are easier to handle. Furthermore, where the fragment is sequenced, the methods will also be cheaper where a shorter sequence is analysed.

Where a fragment is used in the methods of the invention, the fragment should comprise the amino acids corresponding to amino acids 108-116 in SEQ ID NO:2, preferably amino acids 106-118 or amino acids 106-126 in SEQ ID NO:2, because this sequence is the core motif for binding to an MHC class II receptor containing HLA DQB1*0602. Thus, a fragment which can be used with the invention will have a minimal length of 9 amino acids, but as mentioned above will typically be at least around 12 or 13 amino acids in length to bind to an MHC class II receptor, such as at least 18, 19, 20 or 21 amino acids in length. The reference to amino acids 108-116 and 106-118 and 106-126 in SEQ ID NO: 2 in this context does not mean that the invention can be practised only with fragments that comprise the exact same amino acid sequence shown in amino acids 108-116 or 106-118 or 106-126 in SEQ ID NO: 2. Instead, this provides a reference for a skilled person to find the corresponding amino acids in other nucleoprotein sequences (e.g. SEQ ID NO: 3 in strain X-181), thereby identifying the equivalent fragment in any influenza A virus NP.

When a fragment is used from one strain, any comparisons with another strain should be made against the corresponding fragment from that strain e.g. to compare a peptide having SEQ ID NO: 1 with a peptide having SEQ ID NO: 3, rather than with a longer or shorter peptide from strain X-181. The invention can also be practiced with a genome segment encoding an influenza NP protein or a fragment, as described in the preceding paragraphs. Therefore methods can be performed without protein sequencing, but instead by using nucleic acid sequencing (or even using sequence information which has been separately obtained).

The NP protein or the fragment can be analysed by testing its ability to bind to HLA DQB which is a beta-chain subunit of an MHC class II receptor. Accordingly, it will be understood by a person skilled in the art that testing of binding of the NP protein or fragments to HLA DQB1*0602 is based on binding to a functional MHC class II receptor heterodimer where the beta-subunit is HLA DQB1*0602. The alpha subunit will typically be a DQA1 subunit, such as HLA DQA1*0102 (which is the DQA1 subunit most commonly associated with HLA DQB1*0602 in Caucasian Americans). Such heterodimers can be obtained for testing purposes using, for example, recombinant expression technology. A suitable method for expressing soluble MHC class II receptor heterodimers is described in reference 12. The amino acid sequence of HLA DQB1*0602 is available from UniProtKB/Swiss-Prot: P01920). Another suitable method is described in reference 13.

The reference point in these experiments is the NP protein of SEQ ID NO: 2, or more typically a fragment of that sequence as discussed in the previous paragraphs, such as the fragment shown in SEQ ID NO: 1. A NP protein with this sequence has been associated with narcolepsy. A NP protein which binds HLA DQB1*0602 with lower affinity (under the same experimental conditions) compared to this protein is less likely to bind to HLA DQB1*0602 and is thus less likely to cause narcolepsy. As discussed above, typically, the comparison is performed with equivalent fragments. For example, where the NP fragment is the fragment shown in SEQ ID NO: 1, the test NP fragment would be obtained from amino acids 106-126 or the equivalent region, taking into account that different NP proteins may not be of exactly the same length and could have deletions or insertions.

Suitable assays for testing the binding affinity are known in the art e.g. using NMR, filter-binding assays, gel-retardation assays, displacement/competition assays, reverse two-hybrid, surface plasmon resonance and spectroscopy. An example of a suitable peptide-binding assay is described in ref. 12. In a particular example of an assay, a test peptide, such as an NP protein fragment from strain X-179A (such as a fragment consisting of, or comprising, the amino acid sequence shown in SEQ ID NO: 1) is labeled with a detectable label, e.g. biotin) and is bound to the MHC class II receptor containing HLA DQB1*0602 (typically with HLA DQA1*0102 as the other component of the heterodimer). Unlabeled test peptide is then incubated with the receptor-labeled peptide complexes. Whether the test peptide competes successfully for binding with the reference labeled peptide can be determined by measuring the amount of labeled peptide still bound. If the test peptide binds less strongly to the MHC class II receptor containing HLA DQB1*0602 then it will not compete successfully for binding with the labeled reference peptide and therefore this provides a convenient means for identifying NP proteins that bind less strongly to an MHC class II receptor containing HLA DQB1*0602 than the NP protein from strain X-179A.

Other assays are described in reference 13 and reference 88 (*e.g* the REVEAL^{™} ProImmune technology used in the Examples, which measures the ability of synthetic test peptides to stabilize MHC-peptide complexes, where the presence or absence of the native conformation of the MHC-peptide complex, which is detected by a specific monoclonal antibody).

In one embodiment, the NP protein (such as a fragment corresponding to amino acids 106 to 118 of SEQ ID NO: 2 or corresponding to amino acids 106 to 126 of SEQ ID NO:2) that is being tested has at least a two-fold lower binding affinity for an MHC class II receptor containing HLA DQB1*0602 than the NP protein from X-179A (such as a fragment consisting of amino acids 106 to 118 of SEQ ID NO: 2 or consisting of amino acids 106 to 126 of SEQ ID NO:2), such as a three-fold, four-fold, five-fold or ten-fold lower binding affinity for an MHC class II receptor containing HLA DQB1*0602. Preferably the MHC class II receptor being tested is a heterodimer of HLA DQA1*0102 and HLA DQB1*0602.

The NP protein can also be analysed by determining its sequence or the sequence of a fragment, as discussed in the preceding paragraphs. The methods can also be performed by analysing the sequence of the viral segment encoding the NP protein or the fragment thereof. Suitable assays for sequencing peptides and nucleic acids are well known in the art and include, for example Edman degradation assays and Sanger sequencing. Where nucleic acid is analysis, the nucleic acid may be amplified before sequencing, for example by polymerase chain reaction (PCR).

In one embodiment, the analysis is made as to whether the virus's nucleoprotein comprises one or more of (a) an aliphatic amino acid in the position corresponding to amino acid 108 of SEQ ID NO: 2; and/or (b) a aliphatic amino acid in the position corresponding to amino acid 110 of SEQ ID NO: 2; and/or (c) an hydrophobic amino acid in the position corresponding to amino acid 111 of SEQ ID NO: 2. The core binding motifs of the nucleoprotein for binding to HLA DQB1*0602 are at amino acids 108, 110, 111, 113 and 116 of SEQ ID NO: 2, wherein binding works particularly well when amino acids 108 and 110 are aliphatic amino acids and the amino acid at position 111 is a hydrophobic amino acid. Avoiding such amino acids at these positions thus decreases the likelihood that the NP protein can bind to HLA DQB which further decreases the likelihood that the NP protein will cause narcolepsy. An influenza virus is considered particularly suitable for vaccine production if it does not contain one, two or all of these specific amino acids. The binding pocket at position 111 has been shown to be particularly important for binding and it is therefore preferred that the NP protein does not contain a hydrophobic amino acid at this position. It is particularly preferred that the protein does not have tyrosine in this position because known examples of strong binders have this amino acid [8]. The amino acids at positions 108 and/or 110 are preferably not leucine. The influenza virus may also be considered suitable for vaccine production if it does not comprise the sequence LXLYXXXIXXXXXX (SEQ ID NO: 9), wherein X is any amino acid. The protein sequence may also be analysed by alternative means. For example, where a genome segment is analysed, the segments can be analysed using a probe which specifically detects the amino acids which are particularly relevant in the binding motif. Similarly, the NP protein can be analysed using immunochemical methods such as western blot analysis or immunofluorescence. Where such analytical methods are used with the invention, these should be performed using an antibody which allows specific detection of the sequence LILYDKEEI (SEQ ID NO: 8, corresponding to amino acids 108-116 in SEQ ID NO: 2). As discussed above, it is particularly preferred that the NP protein does not have tyrosine in position 111 and the antibody can therefore be an antibody which specifically detects tyrosine in position 111. Alternatively, or in addition the antibody may specifically detect leucine at positions 108 and/or 110. Methods of obtaining antibodies which can distinguish between related sequences are known to those skilled in the art.

### Detecting the nucleoproteins

In some aspects, the invention provides inactivated influenza vaccines which do not comprise an influenza A nucleoprotein. A skilled person will appreciate that it is often not possible to remove all NP from the final vaccine and that a vaccine (especially a split vaccine) will still contain residual amounts of NP. For example, the Focetria™ vaccine still contained a certain amount of NP protein but these proteins could not be a problem because they were present in much lower amounts than they were in Pandemrix™.

Thus, if a vaccine of the invention includes nucleoprotein, it preferably makes up less than 15% by mass of the total influenza virus protein in the vaccine *e.g*. <12%, <10%, <8%, <7%, <6%, <5%, <4%, <3%, <2%, or <1%. The vaccine may comprise less than 3µg NP per 10µg of HA, less than 2.5µg NP per 10µg of HA, less than 2µg NP per 10µg of HA, less than 1.5µ NP per 10µg of HA, less than 1µg NP per 10µg of HA, less than 0.5µg NP per 10µg of HA or less than 0.1µg NP per 10µg of HA. The amount of NP can be determined as described above.

As discussed above, in one embodiment where a mixture of influenza A nucleoproteins are present, it may only be necessary to reduce the amount of the nucleoprotein to below the level specified herein with respect to that nucleoprotein that is to be avoided as per the definitions given in the first aspect of the invention. Thus in this embodiment, the assessment of the amount of NP given above need only be made in relation to such nucleoprotein e.g. the nucleoprotein of strain X-179A. Other nucleoprotein, such as that of strain X-181, may be included at, for example, the usual levels for any particular type of vaccine. Thus, in this embodiment, more stringent purification measures need only be applied during the manufacture of some strains and not others, depending on the sequence/binding characteristics of the particular nucleoprotein. Once combined into the final vaccine composition, the total amount of NP may exceed the limits given above, provided that the amounts of the types of NP that it is desired to reduce do not.

Methods to determine to amount of protein in a composition are known to the skilled person in the art. However, since NP and NA have virtually the same molecular weight (around 60 kDa), they usually co-migrate in non-reducing gels. Classical SDS gel-electrophoresis might therefore not be an appropriate way to determine the amount of NP (see Chaloupka et al., 1996, Eur J Clin Microbiol Infect Dis. 1996 Feb; 15(2): 121-7.). One way to determine the amount ofNP in a vaccine bulk might be a 2 dimensional electrophoresis with a subsequent densitometry. Preferred, however is isotope dilution mass spectrometry using an isotopically labeled synthetic peptide as described in: Williams et al., Vaccine 30 (2012) 2475-2482. This method uses liquid chromatography-tandem mass spectrometry (LC-MS/MS) using isotope dilution in conjunction with multiple reaction monitoring (MRM). This method quantifies targeted peptides released by proteolytic digestion of the sample as a stoichiometric representative of the analyte protein. A stable isotope-labeled reference peptide is spiked into the sample as an internal standard (IS). Quantification of NP is achieved by comparing the peak area of the isotopically labeled reference peptide with that of the endogenous target peptide. This method allows simultaneous quantification of multiple proteins, provided labeled peptides are included for each specific target.

Alternatively, label free mass spectrometry (LC/MSE) is used for the quantification, preferably in quadrupole time-of-flight (Q-Tof) mass spectrometers [11]. For this method, alternating scans of low collision energy and elevated collision energy during LC/MS analysis are used to obtain both protein identity and quantity in a single experiment. Quantification is based on the experimental data showing that the average signal intensity measured by LC/MSE of the three most intense tryptic peptides for any given protein is constant at a given concentration, regardless of protein type and size. As the signal intensity is proportional to concentration, the amount of any protein in the mixture can be estimated.

### The culture host

The influenza viruses are typically produced using a cell line, although primary cells may be used as an alternative. The cell will typically be mammalian, although avian or insect cells can also be used. Suitable mammalian cells include, but are not limited to, human, hamster, cattle, primate and dog cells. In some embodiments, the cell is a human non-kidney cell or a non-human cell. Various cells may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line [14-16]. Suitable dog cells are e.g. kidney cells, as in the CLDK and MDCK cell lines. Suitable avian cells include the EBx cell line derived from chicken embryonic stem cells, EB45, EB14, and EB14-074 [17].

Further suitable cells include, but are not limited to: CHO; MRC 5; PER.C6 [18]; FRhL2; WI-38; *etc.* Suitable cells are widely available *e.g.* from the American Type Cell Culture (ATCC) collection [19], from the Coriell Cell Repositories [20], or from the European Collection of Cell Cultures (ECACC). For example, the ATCC supplies various different Vero cells under catalogue numbers CCL 81, CCL 81.2, CRL 1586 and CRL-1587, and it supplies MDCK cells under catalogue number CCL 34. PER.C6 is available from the ECACC under deposit number 96022940.

Preferred cells for use in the invention are MDCK cells [21-23], derived from Madin Darby canine kidney. The original MDCK cells are available from the ATCC as CCL 34. It is preferred that derivatives of these or other MDCK cells are used. Such derivatives were described, for instance, in reference 21 which discloses MDCK cells that were adapted for growth in suspension culture (`MDCK 33016' or '33016-PF', deposited as DSM ACC 2219). Furthermore, reference 24 discloses MDCK-derived cells that grow in suspension in serum free culture ('B-702', deposited as FERM BP-7449). In some embodiments, the MDCK cell line used may be tumorigenic, but it is also envisioned to use non-tumorigenic MDCK cells. For example, reference 25 discloses non-tumorigenic MDCK cells, including 'MDCK-S'S (ATCC PTA-6500), 'MDCK-SF101' (ATCC PTA-6501), 'MDCK-SF102' (ATCC PTA-6502) and 'MDCK-SF103' (ATCC PTA-6503). Reference 26 discloses MDCK cells with high susceptibility to infection, including 'MDCK.5F1' cells (ATCC CRL 12042).

It is possible to use a mixture of more than one cell type in the methods of the invention, but it is preferred to use a single cell type e.g. using monoclonal cells.

The cells used in the methods of the invention are preferably cells which are suitable for producing an influenza vaccine that can be used for administration to humans. Such cells must be derived from a cell bank system which is approved for vaccine manufacture and registered with a national control authority, and must be within the maximum number of passages permitted for vaccine production (see reference 27 for a summary). Examples of suitable cells which have been approved for vaccine manufacture include MDCK cells (like MDCK 33016; see reference 21), CHO cells, Vero cells, and PER.C6 cells. The methods of the invention preferably do not use 293T cells as these cells are not approved for vaccine manufacture.

Preferably, the cells used for preparing the virus and for preparing the vaccine are of the same cell type. For example, the cells may both be MDCK, Vero or PerC6 cells. This is preferred because it facilitates regulatory approval as approval needs to be obtained only for a single cell line. It also has the further advantage that competing culture selection pressures or different cell culture conditions can be avoided. The methods of the invention may also use the same cell line throughout, for example MDCK 33016.

The influenza viruses may also be propagated in eggs. The current standard method for influenza virus growth for vaccines uses embryonated SPF hen eggs, with virus being purified from the egg contents (allantoic fluid). It is also possible to passage a virus through eggs and subsequently propagate it in cell culture and *vice versa.*

### Virus preparation

The invention provides a method of preparing an influenza virus, comprising the steps of (a) testing the suitability of the influenza virus for vaccine production by the methods discussed above; (b) infecting a culture host with the influenza virus of step (a); and (c) culturing the host from step (b) to produce further virus; and, optionally (d) purifying virus obtained in step (c).

The culture host may be cells or embryonated hen eggs, as discussed in the previous paragraphs. Where cells are used as a culture host in this aspect of the invention, it is known that cell culture conditions (e.g. temperature, cell density, pH value, etc.) are variable over a wide range subject to the cell line and the virus employed and can be adapted to the requirements of the application. The following information therefore merely represents guidelines.

Preferably, the cells are cultured in the absence of serum, to avoid a common source of contaminants. Various serum-free media for eukaryotic cell culture are known to the person skilled in the art *e.g.* Iscove's medium, ultra CHO medium (BioWhittaker), EX-CELL (JRH Biosciences). Furthermore, protein-free media may be used *e.g*. PF-CHO (JRH Biosciences). Otherwise, the cells for replication can also be cultured in the customary serum-containing media (*e.g.* MEM or DMEM medium with 0.5% to 10% of fetal calf serum).

Multiplication of the cells can be conducted in accordance with methods known to those of skill in the art. For example, the cells can be cultivated in a perfusion system using ordinary support methods like centrifugation or filtration. Moreover, the cells can be multiplied according to the invention in a fed-batch system before infection. In the context of the present invention, a culture system is referred to as a fed-batch system in which the cells are initially cultured in a batch system and depletion of nutrients (or part of the nutrients) in the medium is compensated by controlled feeding of concentrated nutrients. It can be advantageous to adjust the pH value of the medium during multiplication of cells before infection to a value between pH 6.6 and pH 7.8 and especially between a value between pH 7.2 and pH 7.3. Culturing of cells preferably occurs at a temperature between 30 and 40°C. When culturing the infected cells (step c), the cells are preferably cultured at a temperature of between 30°C and 36°C or between 32°C and 34°C or at 33°C. This is particularly preferred, as it has been shown that incubation of infected cells in this temperature range results in production of a virus that results in improved efficacy when formulated into a vaccine [28].

Oxygen partial pressure can be adjusted during culturing before infection preferably at a value between 25% and 95% and especially at a value between 35% and 60%. The values for the oxygen partial pressure stated in the context of the invention are based on saturation of air. Infection of cells occurs at a cell density of preferably about 8-25x10⁵ cells/mL in the batch system or preferably about 5-20x10⁶ cells/mL in the perfusion system. The cells can be infected with a viral dose (MOI value, "multiplicity of infection"; corresponds to the number of virus units per cell at the time of infection) between 10⁻⁸ and 10, preferably between 0.0001 and 0.5.

Virus may be grown on cells in adherent culture or in suspension. Microcarrier cultures can be used. In some embodiments, the cells may thus be adapted for growth in suspension.

The methods according to the invention also include harvesting and isolation of viruses or the proteins generated by them. During isolation of viruses or proteins, the cells are separated from the culture medium by standard methods like separation, filtration or ultrafiltration. The viruses or the proteins are then concentrated according to methods sufficiently known to those skilled in the art, like gradient centrifugation, filtration, precipitation, chromatography, etc., and then purified. It is also preferred according to the invention that the viruses are inactivated during or after purification. Virus inactivation can occur, for example, by β-propiolactone or formaldehyde at any point within the purification process.

### Vaccine

Influenza vaccines are generally based either on live virus or on inactivated virus. Inactivated vaccines are preferred with the present invention, and these may be based on whole virions, 'split' virions, or on purified surface antigens. Antigens can also be presented in the form of virosomes. The invention can be used for manufacturing any of these types of vaccine. It is particularly suitable for manufacturing influenza vaccines, however, which generally comprise nucleoprotein. Such influenza vaccines include live virus, whole virion or split virion influenza vaccines. The vaccines encompassed by the present invention are those for which nucleoprotein is present during one or more process/manufacturing steps, and therefore could conceivably contain nucleoprotein in the final vaccine composition which could increase the risk of an autoimmune response in susceptible individuals unless steps are taken to reduce or avoid binding of the NP to particular MHC class II receptor subtypes.

In the first aspect of invention, the vaccines of the present invention have influenza A NP proteins wherein the NP proteins have (or fragments of them have) a lower binding affinity for HLA DQB1*0602 than the NP protein of strain X-179A (or a fragment therefore such as a fragment consisting or comprising amino acids 106 or 108 to 118 or 120 or 126 of the sequence shown in SEQ ID NO: 2). Expressed the other way, the NP proteins of such vaccines lack regions that bind to HLA DQB1*0602 with the same or higher affinity as the NP protein of strain X-179A (or a fragment therefore such as a fragment consisting or comprising amino acids 106 or 108 to 118 or 120 or 126 of the sequence shown in SEQ ID NO: 2).

In the second aspect of the invention, the vaccines of the present invention have influenza A NP proteins that lack an isoleucine at a position corresponding to amino acid residue 116 of SEQ ID NO:2. For example, the influenza A NP proteins present in the vaccine composition

It will clear, as discussed earlier, that since the intention is to avoid the presence of NP that could exhibit significant binding to an MHC class II receptor including HLA DQB 1*0602 then reference to influenza A nucleoprotein in the compositions of the invention must consider the total influenza A nucleoprotein in the composition.Where an inactivated virus is used, the vaccine may comprise whole virion, split virion, or purified surface antigens (for influenza, including hemagglutinin and, usually, also including neuraminidase). Chemical means for inactivating a virus include treatment with an effective amount of one or more of the following agents: detergents, formaldehyde, β-propiolactone (BPL), methylene blue, psoralen, carboxyfullerene (C60), binary ethylamine, acetyl ethyleneimine, or combinations thereof. Non-chemical methods of viral inactivation are known in the art, such as for example UV light or gamma irradiation. It is also possible to inactivate the influenza viruses using a combination of different methods. For example, a combination of BPL and UV light is useful.

So far narcolepsy cases have only been detected at a higher rate than background incidence in subjects vaccinated with the Dresden antigen of Pandemrix™ (see below). The Dresden antigen contains Triton and Tween. To reduce the risk of narcolepsy derived from flu vaccines which contain an oil-in-water emulsion adjuvant, and an antigen containing Tween and Triton X-100 detergent, two ways are conceivable: (i) use an antigen component without NP or with a substantially reduced amount of NP, like in a subunit vaccine, or (ii) use of a seed virus for the vaccine production which does not contain NP, or a fragment thereof, which can bind to HLA DQB 1*0602.

Thus, one aspect of the invention is an oil-in-water adjuvanted, inactivated influenza vaccine which does not contain influenza A NP protein, or contains less than 15% by mass of the total influenza virus protein in the vaccine *e*.*g*. <12%, <10%, <8%, <7%, <6%, <5%, <4%, <3%, <2%, or <1%. In one embodiment the adjuvant contains an additional immunopotentiator tocopherol, GLA or a TLR agonist. In a preferred embodiment the adjuvant is AS03. In one embodiment the virus is formaldehyde inactivated. In one embodiment the vaccine contains thiomersal. In a preferred embodiment the antigen component of the vaccine contains a detergent, in particular Tween 80 and/or Triton x-100 (t- octylphenoxypolyethoxyethanol). Preferably the weight/volume ratio between Triton X-100 and HA is between 1.5 and 15. Preferably the Triton-100™ concentration is between 10 and 500 µg/ml. In a particular preferred embodiment the vaccine is a purified sub-unit vaccine adjuvanted with AS03. The antigen and the adjuvant component of the vaccine might be premixed or might be in separate containers for mixture by the end-user/health care provider before administration. One aspect of the invention is the use of the antigen component specified above for formulation with an oil-in-water adjuvant, in particular with AS03.

Another aspect of the invention is an AS03 adjuvanted, inactivated, split influenza A vaccine which does contain NP protein, but which does not contain NP protein or NP fragments that can bind to HLA DQB1*0602. This can be achieved if a backbone is used which is similar to that used in Foecetria (X-181), but which is different from that in Pandemrix™. In a preferred embodiment the vaccine is against a H1, H3, H5, H7 or H9 strain, in a particular preferred embodiment against a pandemic H1, H3, H5, H7 or H9 strain. In one embodiment the virus is formaldehyde inactivated. In one embodiment the vaccine contains thiomersal; in an alternative embodiment it is preservative-free. The antigen component of the vaccine contains a detergent, in particular Tween 80 and/or Triton x-100 (t- octylphenoxypolyethoxyethanol). Preferably the weight/volume ratio between Triton X-100 and HA is between 1.5 and 15. Preferably the Triton-100™ concentration is between 10 and 500 µg/ml. The vaccine might contain the excipients as shown in the table WO2011/051235 (see below) in similar or identical concentration. The antigen and the adjuvant component of the vaccine might be premixed or might be in separate containers for mixture by the end-user/health care provider before administration. One aspect of the invention is the use of the antigen component specified above for formulation with an oil-in-water adjuvant, in particular with AS03.

Another aspect of the invention is an AS03 adjuvanted, inactivated, split influenza A vaccine which contains NP protein that binds to HLA DQB 1*0602 with lower affinity under the same conditions compared to nucleoprotein from strain X-179A. In a preferred embodiment the vaccine is against a H1, H3, H5, H7 or H9 strain, in a particular preferred embodiment against a pandemic H1, H3, H5 H7 or H9 strain The antigen component of the vaccine contains a detergent, in particular Tween 80 and/or Triton X-100. The vaccine might contain the excipients as shown in the table from WO2011/051235 (see below) in similar or identical concentration. The antigen and the adjuvant component of the vaccine might be premixed or might be in separate containers for mixture by the end-user/health care provider before administration. One aspect of the invention is the use of the antigen component specified above for the formulation with an oil-in-water adjuvant, in particular with AS03.

Another aspect of the invention is an AS03 adjuvanted, inactivated, split influenza A vaccine which contains NP protein does not contain isoleucine in the position corresponding to amino acid 116 of SEQ ID NO: 2 (see Figure 1). In a preferred embodiment the vaccine is against a H1, H3, H5, H7 or H9 strain, in a particular preferred embodiment against a pandemic H1, H3, H5, H7 or H9 strain. The antigen component of the vaccine contains a detergent, in particular Tween 80 and/or Triton X-100. The vaccine might contain the excipients as shown in the table WO2011/051235 (see below) in similar or identical concentration. The antigen and the adjuvant component of the vaccine might be premixed or might be in separate containers for mixture by the end-user/health care provider before administration. One aspect of the invention is the use of the antigen component specified above for the formulation with an oil-in-water adjuvant, in particular with AS03.

Another aspect of the invention is an AS03 adjuvanted, inactivated, split influenza A vaccine which contains a NP protein which does not contain the sequence LXLYXXXIXXXXXX (SEQ ID NO: 9), wherein X is any amino acid. In a preferred embodiment the vaccine is against a H1, H3, H5, H7 or H9 strain, in a particular preferred embodiment against a pandemic H1, H3, H5, H7 or H9 strain. The vaccine might contain the excipients as shown in the table WO2011/051235 (see below) in similar or identical concentration. The antigen and the adjuvant component of the vaccine might be premixed or might be in separate containers for mixture by the end-user/health care provider before administration. One aspect of the invention is the use of the antigen component specified above for the formulation with an oil-in-water adjuvant, in particular with AS03.

Virions can be harvested from virus-containing fluids, *e.g*. allantoic fluid or cell culture supernatant, by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g*. ethyl ether, polysorbate 80, deoxycholate, tri-*N*-butyl phosphate, Triton X-100, Triton N101, cetyltrimethylammonium bromide, Tergitol NP9, *etc.*) to produce subvirion preparations, including the 'Tween-ether' splitting process. Methods of splitting influenza viruses, for example are well known in the art *e.g.* see refs. 29-34, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, NP9, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g*. the Triton surfactants, such as Triton X-100 or Triton N101), polyoxyethylene sorbitan esters (the Tween surfactants), polyoxyethylene ethers, polyoxyethylene esters, *etc.* One useful splitting procedure uses the consecutive effects of sodium deoxycholate and formaldehyde, and splitting can take place during initial virion purification (e.g. in a sucrose density gradient solution). Thus a splitting process can involve clarification of the virion-containing material (to remove non-virion material), concentration of the harvested virions (*e.g*. using an adsorption method, such as CaHPO₄ adsorption), separation of whole virions from non-virion material, splitting of virions using a splitting agent in a density gradient centrifugation step (*e.g*. using a sucrose gradient that contains a splitting agent such as sodium deoxycholate), and then filtration (*e.g*. ultrafiltration) to remove undesired materials. Split virions can usefully be resuspended in sodium phosphate-buffered isotonic sodium chloride solution. Examples of split influenza vaccines are the BEGRIVAC™, FLUARIX™, FLUZONE™ and FLUSHIELD™ products.

Purified influenza virus surface antigen (glycoprotein) vaccines comprise the surface antigens hemagglutinin and, typically, also neuraminidase. Processes for preparing these proteins in purified form are well known in the art. The FLUVIRIN™, AGRIPPAL™ and INFLUVAC™ products are influenza subunit vaccines. Purified surface glycoprotein vaccines can be particularly advantageous relative to split vaccines because they include much lower levels of NP protein.

Another form of inactivated antigen is the virosome [35] (nucleic acid free viral-like liposomal particles). Virosomes can be prepared by solubilization of virus with a detergent followed by removal of the nucleocapsid and reconstitution of the membrane containing the viral glycoproteins. An alternative method for preparing virosomes involves adding viral membrane glycoproteins to excess amounts of phospholipids, to give liposomes with viral proteins in their membrane.

The methods of the invention may also be used to produce live vaccines. Such vaccines are usually prepared by purifying virions from virion-containing fluids. For example, the fluids may be clarified by centrifugation, and stabilized with buffer (*e.g*. containing sucrose, potassium phosphate, and monosodium glutamate). Various forms of live attenuated influenza virus vaccine are currently available (e.g. see chapters 17 & 18 of ref. 36). Live vaccines include the FLUMIST™ products.

Where the invention concerns a live vaccine, references to the presence or absence of a particular influenza virus A nucleoprotein in a composition can apply also to the nucleoprotein which is encoded by the strains within the vaccine, as well as to the nucleoprotein in the vaccine itself. For instance, the influenza A strains may encode nucleoproteins in which a fragment equivalent to amino acids 106 to 118 of SEQ ID NO: 2 binds to an MHC class II receptor comprising HLA DQB 1*0602 with a lower affinity than a peptide having the amino acid sequence shown in SEQ ID NO: 1. Like X-179A, the A/Ann Arbor/6/60 backbone has isoleucine at position 116 (see AY210074), and so a useful live vaccine of the invention includes an influenza A virus strain which encodes a NP having a residue other than isoleucine at position 116 *e.g.* a methionine.

The virus may be attenuated. The virus may be temperature-sensitive. The virus may be cold-adapted. These three features are particularly useful when using live virus as an antigen.

HA is the main immunogen in current inactivated influenza vaccines, and vaccine doses are standardised by reference to HA levels, typically measured by SRID. Existing vaccines typically contain about 15µg of HA per strain, although lower doses can be used *e.g.* for children, or in pandemic situations, or when using an adjuvant. Fractional doses such as ½ (*i.e.* 7.5µg HA per strain), ¼ and ⅛ have been used, as have higher doses *(e.g.* 3x or 9x doses [37,38]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.8, about 3.75, about 1.9, about 1.5, *etc.* per strain. In preferred embodiments, the vaccine includes HA at a concentration of <30µg/ml. it may also comprise HA at a concentration of <10µg per unit dose, 7.5µg per unit dose; <5µg per unit dose; or 3.75µg per unit dose.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical.

Influenza strains used with the invention may have a natural HA as found in a wild-type virus, or a modified HA. For instance, it is known to modify HA to remove determinants (*e.g*. hyper-basic regions around the HA1/HA2 cleavage site) that cause a virus to be highly pathogenic in avian species. The use of reverse genetics facilitates such modifications.

As well as being suitable for immunizing against inter-pandemic strains, the compositions of the invention are particularly useful for immunizing against pandemic or potentially-pandemic strains. The invention is suitable for vaccinating humans as well as non-human animals.

Other strains whose antigens can usefully be included in the compositions are strains which are resistant to antiviral therapy (*e.g*. resistant to oseltamivir [39] and/or zanamivir), including resistant pandemic strains [40].

Compositions of the invention may include antigen(s) from one or more (*e.g.* 1, 2, 3, 4 or more) influenza virus strains, including influenza A virus and/or influenza B virus. Where a vaccine includes more than one strain of influenza, the different strains are typically grown separately and are mixed after the viruses have been harvested and antigens have been prepared. Thus a process of the invention may include the step of mixing antigens from more than one influenza strain. A trivalent vaccine is typical, including antigens from two influenza A virus strains and one influenza B virus strain. A tetravalent vaccine is also useful [41], including antigens from two influenza A virus strains and two influenza B virus strains, or three influenza A virus strains and one influenza B virus strain.

An influenza vaccine may have antigens only from seasonal influenza strains, or may have antigens only from a pandemic strain (monovalent). Thus the vaccine composition may be a monovalent pandemic with one A strain only. It may also have antigens from both seasonal and pandemic influenza strains. For example, the vaccine may be a tetravalent vaccine comprising antigens from three seasonal influenza strains (for example, two A and one B strain) and a pandemic influenza strain. A trivalent seasonal influenza vaccine may also be co-administered with a monovalent pandemic influenza vaccine.

In one preferred embodiment the vaccine includes antigen from 4 or more influenza virus strains. In a particularly preferred embodiment the vaccine contains 2 A and 2 B strains, for example (i) a A/H1N1 strain; (ii) an A/H3N2 strain; (iii) a B/Victoria/2/87-like strain; and (iv) B/Yamagata/ 16/88-like strain. In another particularly preferred embodiment one of the strains is a pandemic strain like a H5N1 or a H7N9 strain, e.g. in a combination with (i) a A/H1N1 strain; (ii) a A/H3N2 strain; and (iii) a B strain. The 4 or higher-valent vaccines might be adjuvanted.

Influenza A virus currently displays eighteen HA subtypes: H1, H2, H3, H4, H5, H6, H7, H8, H9, H10, H11, H12, H13, H14, H15, H16, H17, and H18. It currently has nine NA subtypes N1, N2, N3, N4, N5, N6, N7, N8 and N9. In vaccines including two influenza A virus strains, these will usually be from different HA subtypes (*e.g.* H1 and H3) and different NA subtypes (*e.g.* N1 and N2), so a vaccine can include antigens from *e.g.* a H1N1 strain and a H3N2 strain.

Influenza B virus currently does not display different HA subtypes, but influenza B virus strains do fall into two distinct lineages. These lineages emerged in the late 1980s and have HAs which can be antigenically and/or genetically distinguished from each other [42].Where a vaccine of the invention includes antigens from two influenza B strains, these will usually be one B/Victoria/2/87-like strain and one B/Yamagata/16/88-like strain. These strains are usually distinguished antigenically, but differences in amino acid sequences have also been described for distinguishing the two lineages e.g. B/Yamagata/16/88-like strains often (but not always) have HA proteins with deletions at amino acid residue 164, numbered relative to the 'Lee40' HA sequence [43].

### Pharmaceutical compositions

Vaccine compositions manufactured according to the invention are pharmaceutically acceptable. They usually include components in addition to the antigens e.g. they typically include one or more pharmaceutical carrier(s) and/or excipient(s). As described below, adjuvants may also be included. A thorough discussion of such components is available in reference 44.

Vaccine compositions will generally be in aqueous form. However, some vaccines may be in dry form, e.g. in the form of injectable solids or dried or polymerized preparations on a patch.

Vaccine compositions may include preservatives such as thiomersal or 2-phenoxyethanol. It is preferred, however, that the vaccine should be substantially free from (*i.e.* less than 5µg/ml) mercurial material *e.g*. thiomersal-free [33,45]. Vaccines containing no mercury are more preferred. An α-tocopherol succinate can be included as an alternative to mercurial compounds [33]. Preservative-free vaccines are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Vaccine compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [46], but keeping osmolality in this range is nevertheless preferred.

Vaccine compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a vaccine composition will generally be between 5.0 and 8.1, and more typically between 6.0 and 8.0 *e.g.* 6.5 and 7.5, or between 7.0 and 7.8. A process of the invention may therefore include a step of adjusting the pH of the bulk vaccine prior to packaging.

The vaccine composition is preferably sterile. The vaccine composition is preferably non-pyrogenic e.g. containing <1 EU (endotoxin unit, a standard measure) per dose, and preferably <0.1 EU per dose. The vaccine composition is preferably gluten-free.

Vaccine compositions of the invention may include detergent *e.g.* a polyoxyethylene sorbitan ester surfactant (known as 'Tweens's), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may include less than 1mg/ml of each of octoxynol-10 and polysorbate 80. Other residual components in trace amounts could be antibiotics (*e.g*. neomycin, kanamycin, polymyxin B).

A vaccine composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume (unit dose) of about 0.5ml, although a half dose (*i.e*. about 0.25ml) may be administered to children.

Compositions and kits are preferably stored at between 2°C and 8°C. They should not be frozen. They should ideally be kept out of direct light.

### Host cell DNA

Where virus has been isolated and/or grown on a cell line, it is standard practice to minimize the amount of residual cell line DNA in the final vaccine, in order to minimize any potential oncogenic activity of the DNA.

Thus a vaccine composition prepared according to the invention preferably contains less than 10ng (preferably less than 1ng, and more preferably less than 100pg) of residual host cell DNA per dose, although trace amounts of host cell DNA may be present.

It is preferred that the average length of any residual host cell DNA is less than 500bp *e.g.* less than 400bp, less than 300bp, less than 200bp, less than 100bp, *etc.*

Contaminating DNA can be removed during vaccine preparation using standard purification procedures *e.g.* chromatography, *etc.* Removal of residual host cell DNA can be enhanced by nuclease treatment *e.g*. by using a DNase. A convenient method for reducing host cell DNA contamination is disclosed in references 47 & 48, involving a two-step treatment, first using a DNase (*e.g.* Benzonase), which may be used during viral growth, and then a cationic detergent (*e.g.* CTAB), which may be used during virion disruption. Treatment with an alkylating agent, such as β-propiolactone, can also be used to remove host cell DNA, and advantageously may also be used to inactivate virions [49]. Where a DNase or an alkylating agent is used for DNA removal, the DNase or the alkylating agent (preferably BPL) may also be added more than once (for example twice) during the production process. DNA removal may also be accomplished using a combination of a DNase and an alkylating agent.

### Adjuvants

Compositions of the invention may advantageously include an adjuvant, which can function to enhance the immune responses (humoral and/or cellular) elicited in a subject who receives the composition. Preferred adjuvants comprise oil-in-water emulsions. Various such adjuvants are known, and they typically include at least one oil and at least one surfactant, with the oil(s) and surfactant(s) being biodegradable (metabolisable) and biocompatible. The oil droplets in the emulsion are generally less than 5µm in diameter, and ideally have a sub-micron diameter, with these small sizes being achieved with a microfluidiser to provide stable emulsions. Droplets with a size less than 220nm are preferred as they can be subjected to filter sterilization.

The emulsion can comprise oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art.

Another preferred oil for some embodiments is α-tocopherol. D-α-tocopherol and DL-α-tocopherol can both be used, and the preferred α-tocopherol is DL-α-tocopherol. The tocopherol can take several forms *e.g*. different salts and/or isomers. Salts include organic salts, such as succinate, acetate, nicotinate, *etc.* If a salt of this tocopherol is to be used, the preferred salt is the succinate. An oil combination comprising squalene and a tocopherol (*e.g*. DL-α-tocopherol) can be used, as seen in the AS03 adjuvant. As explained above, however, in some embodiments an emulsion does not include any additional immunostimulating agents, in which case the emulsion would not include α-tocopherol.

Oil-in-water emulsions including squalene are the most preferred adjuvants for use with the invention *e.g*. MF59 or AS03 (or a modified AS03 which lacks tocopherol). Thus a preferred emulsion can consist essentially of (i) water or buffer, (ii) squalene, and (iii) polysorbate 80 and/or sorbitan trioleate.

Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used e.g. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1% or about 0.5%.

Where the vaccine contains a split virus, it is preferred that it contains free surfactant in the aqueous phase. This is advantageous as the free surfactant can exert a 'splitting effect' on the antigen, thereby disrupting any unsplit virions and/or virion aggregates that might otherwise be present. The free surfactant can further prevent aggregation of any unsplit virions which may be present. This can improve the safety of split virus vaccines [50].

Preferred emulsions have an average droplets size of <1µm e.g. ≤750nm, ≤500nm, ≤400nm, ≤300nm, ≤250nm, ≤220nm, ≤200nm, or smaller. These droplet sizes can conveniently be achieved by techniques such as microfluidisation.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [51-53], as described in more detail in Chapter 10 of ref. 54 and chapter 12 of ref. 55. The MF59 emulsion advantageously includes citrate ions e.g. 10mM sodium citrate buffer.
- An emulsion comprising squalene, a tocopherol, and polysorbate 80. The emulsion may include phosphate buffered saline. These emulsions may have by volume from 2 to 10% squalene, from 2 to 10% tocopherol and from 0.3 to 3% polysorbate 80, and the weight ratio of squalene:tocopherol is preferably <1 *(e.g.* 0.90) as this can provide a more stable emulsion. Squalene and polysorbate 80 may be present volume ratio of about 5:2 or at a weight ratio of about 11:5. Thus the three components (squalene, tocopherol, polysorbate 80) may be present at a weight ratio of 1068:1186:485 or around 55:61:25. One such emulsion ('AS03' [56]) has 4.86 mg polysorbate 80, 10.69 mg squalene and 11.86 mg α-tocopherol per dose (or a fraction thereof, but maintaining the mass ratios) *e.g*. in a 0.5ml volume. AS03 can be made by dissolving Tween 80 in PBS to give a 2% solution, then mixing 90ml of this solution with a mixture of (5g of DL α tocopherol and 5ml squalene), then microfluidising the mixture. The resulting emulsion may have submicron oil droplets *e.g*. with an average diameter of between 100 and 250nm, preferably about 180nm. The emulsion may also include a 3-de-O-acylated monophosphoryl lipid A (3d MPL). Another useful emulsion of this type may comprise, per human dose, 0.5-10 mg squalene, 0.5-11 mg tocopherol, and 0.1-4 mg polysorbate 80 [57] *e.g.* in the ratios discussed above.

- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate (*e.g.* polysorbate 80), a Triton detergent (*e.g.* Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [58] (0.05-1% Thr-MDP, 5% squalane, 2.5% Pluronic L121 and 0.2% polysorbate 80). It can also be used without the Thr-MDP, as in the "AF" adjuvant [59] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [60]. The emulsion may also include one or more of: alditol; a cryoprotective agent (*e.g.* a sugar, such as dodecylmaltoside and/or sucrose); and/or an alkylpolyglycoside. The emulsion may include a TLR4 agonist [61]. Such emulsions may be lyophilized.
- An emulsion of squalene, poloxamer 105 and Abil-Care [62]. The final concentration (weight) of these components in adjuvanted vaccines are 5% squalene, 4% poloxamer 105 (pluronic polyol) and 2% Abil-Care 85 (Bis-PEG/PPG-16/16 PEG/PPG-16/16 dimethicone; caprylic/capric triglyceride).
- An emulsion having from 0.5-50% of an oil, 0.1-10% of a phospholipid, and 0.05-5% of a non-ionic surfactant. As described in reference 63, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 64, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g*. QuilA or QS21) and a sterol (*e.g.* a cholesterol) are associated as helical micelles [65].
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [66].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [66].

In some embodiments an emulsion may be mixed with antigen extemporaneously, at the time of delivery, and thus the adjuvant and antigen may be kept separately in a packaged or distributed vaccine, ready for final formulation at the time of use. In other embodiments an emulsion is mixed with antigen during manufacture, and thus the composition is packaged in a liquid adjuvanted form. The antigen will generally be in an aqueous form, such that the vaccine is finally prepared by mixing two liquids. The volume ratio of the two liquids for mixing can vary (*e.g*. between 5:1 and 1:5) but is generally about 1:1. Where concentrations of components are given in the above descriptions of specific emulsions, these concentrations are typically for an undiluted composition, and the concentration after mixing with an antigen solution will thus decrease.

Compositions of the invention might include an additional immunostimulating agent. In a preferred embodiment, the additional immunostimulating agent is a TLR agonist *i.e.* a compound which can agonise a Toll-like receptor. Most preferably, a TLR agonist is an agonist of a human TLR. The TLR agonist can activate any of TLR1, TLR2, TLR3, TLR4, TLR5, TLR6, TLR7, TLR8, TLR9 or TLR11; preferably it can activate human TLR4 or human TLR7.

A composition of the invention can include more than one TLR agonist. These two agonists are different from each other and they can target the same TLR or different TLRs.

Agonist activity of a compound against any particular Toll-like receptor can be determined by standard assays. Companies such as Imgenex and Invivogen supply cell lines which are stably co-transfected with human TLR genes and NFκB, plus suitable reporter genes, for measuring TLR activation pathways. They are designed for sensitivity, broad working range dynamics and can be used for high-throughput screening. Constitutive expression of one or two specific TLRs is typical in such cell lines. Many TLR agonists are known in the art *e.g*. US-4,666,886 describes certain lipopeptide molecules that are TLR2 agonists, WO2009/118296, WO2008/005555, WO2009/111337 and WO2009/067081 each describe classes of small molecule agonists of TLR7, and WO2007/040840 and WO2010/01 describe TLR7 and TLR8 agonists for treatment of diseases.

TLR7 agonists which can be used with the invention can be benzonaphthyridines, such as those having formula T1: 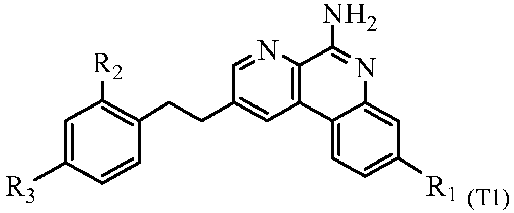 where
R¹ is H, C₁-C₆alkyl, C(R⁵)₂OH, -L¹R⁵, -L¹R⁶, -L²R⁵, -L²R⁶, -OL²R⁵, or -OL²R⁶;
L¹ is -C(O)- or -O-;
L² is C₁-C₆alkylene, C₂-C₆alkenylene, arylene, heteroarylene or -((CR⁴R⁴)ₚO)_{q}(CH₂)ₚ-, wherein the C₁-C₆alkylene and C₂-C₆alkenylene of L² are optionally substituted with 1 to 4 fluoro groups;
each L³ is independently selected from C₁-C₆alkylene and -((CR⁴R⁴)ₚO)_{q}(CH₂)ₚ-, wherein the C₁-C₆alkylene of L³ is optionally substituted with 1 to 4 fluoro groups;
L⁴ is arylene or heteroarylene;
R² is H or C₁-C₆alkyl;
R³ is selected from C₁-C₄alkyl, -L³R⁵, -L¹R⁵, -L³R⁷, -L³L⁴L³R⁷, -L³L⁴R⁵, -L³L⁴L³R⁵, -OL³R⁵, -OL³R⁷, -OL³L⁴R⁷, -OL³L⁴L³R⁷, -OR⁸, -OL³L⁴R⁵, -OL³L⁴L³R⁵ and - C(R⁵)₂OH;
each R⁴ is independently selected from H and fluoro;
R⁵ is -P(O)(OR⁹)₂,
R⁶ is -CF₂P(O)(OR⁹)₂ or -C(O)OR¹⁰;
R⁷ is -CF₂P(O)(OR⁹)₂ or -C(O)OR¹⁰;
R⁸ is H or C₁-C₄alkyl;
each R⁹ is independently selected from H and C₁-C₆alkyl;
R¹⁰ is H or C₁-C₄alkyl;
each p is independently selected from 1, 2, 3, 4, 5 and 6, and
q is 1, 2, 3 or 4.

Further details of these compounds are disclosed in WO2011/049677, and the invention can use any of compounds 1 to 28 therein. Preferred examples of compounds of formula T1 include:

| | |
|---|---|
| T1a | 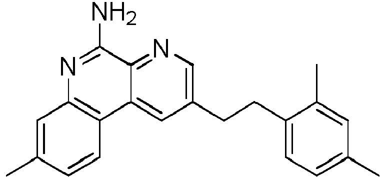 |
| T1b | 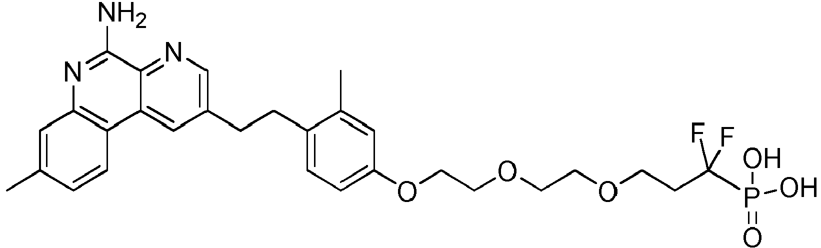 |
| T1c | 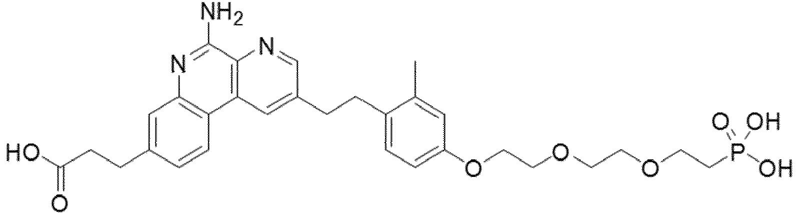 |

Other useful TLR7 agonists include, but are not limited to, or any of compounds 1 to 247 disclosed in WO2009/111337, or any of compounds 1 to 102 from WO2012/031140.

TLR2 agonists which can be used with the invention can be lipopeptides having formula T2: 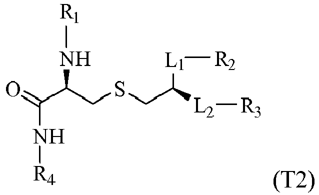 wherein:
- R¹: is H, -C(O)-C₇-C₁₈alkyl or -C(O)- C₁-C₆alkyl;
- R²: is C₇-C₁₈alkyl ;
- R³: is C₇-C₁₈alkyl;
- Lᵢ: is -CH₂OC(O)-, -CH₂O-, -CH₂NR⁷C(O)- or -CH₂OC(O)N_{R}⁷-;
- L₂: is -OC(O)-, -O-, -Nk⁷C(O)- or -OC(O)NR⁷-;
- R⁴: is -L₃R⁵ or -L₄R⁵;
- R⁵: is -N(R⁷)₂, -OR⁷, -P(O)(OR⁷)₂, -C(O)OR⁷, -NR⁷C(O)L₃R⁸, -NR⁷C(O)L₄R⁸, -OL₃R⁶, - C(O)NR⁷L₃R⁸, -C(O)NR⁷L₄R⁸, -S(O)₂OR⁷, -OS(P)₂OR⁷, C₁-C₆alkyl, a C₆aryl, a C₁₀aryl, a C₁₄aryl, 5 to 14 ring membered heteroaryl containing 1 to 3 heteroatoms selected from O, S and N, C₃-C₈cycloalkyl or a 5 to 6 ring membered heterocycloalkyl containing 1 to 3 heteroatoms selected from O, S and N, wherein the aryl, heteroaryl, cycloalkyl and heterocycloalkyl of R⁵ are each unsubstituted or the aryl, heteroaryl, cycloalkyl and heterocycloalkyl of R⁵ are each substituted with 1 to 3 substituents independently selected from -OR⁹, -OL₃R⁶, -OL₄R⁶, -OR⁷, and - C(O)OR⁷;
- L³: is a C₁-C₁₀alkylene, wherein the C₁-C₁₀alkylene of L³ is unsubstituted, or the C₁-C₁₀alkylene of L³ is substituted with 1 to 4 R⁶ groups, or the C₁-C₁₀alkylene of L³ is substituted with 2 C₁-C₆alkyl groups on the same carbon atom which together, along with the carbon atom they are attached to, form a C₃-C₈cycloakyl;
- L₄: is -((CR⁷R⁷)ₚO)_{q}(CR¹⁰R¹⁰)ₚ- or -(CR¹¹R¹¹)((CR⁷R⁷)ₚO)_{q}(CR¹⁰R¹⁰)ₚ, wherein each R¹¹ is a C₁-C₆alkyl groups which together, along with the carbon atom they are attached to, form a C₃-C₈cycloakyl;
- each R⁶: is independently selected from halo, C₁-C₆alkyl, C₁-C₆alkyl substituted with 1-2 hydroxyl groups, -OR⁷, -N(R⁷)₂, -C(O)OH, -C(O)N(R⁷)₂, -P(O)(OR⁷)₂, a C₆aryl, a C₁₀aryl and a C₁₄aryl;
- each R⁷: is independently selected from H and C₁-C₆alkyl;
- R⁸: is selected from -SR⁷, -C(O)OH, -P(O)(OR⁷)₂, and a 5 to 6 ring membered heterocycloalkyl containing 1 to 3 heteroatoms selected from O and N;
- R⁹: is phenyl;

each R¹⁰ is independently selected from H and halo;
each p is independently selected from 1, 2, 3, 4, 5 and 6, and
q is 1, 2, 3 or 4.

Further details of these compounds are disclosed in WO2011/119759, and the invention can use any of the compounds disclosed therein *e.g*. examples 1-92 thereof, and the compounds listed in claim 17 thereof. Another useful TLR2 agonist is palmitoyl-Cys(2[R],3-dilauroyloxy-propyl)-Abu-D-Glu-NH₂, where: Cys is a cysteine residue, Abu is an aminobutyric acid residue and Glu is a glutamic acid residue. This compound is disclosed in example 16 of US-4,666,886, and has formula T3a:

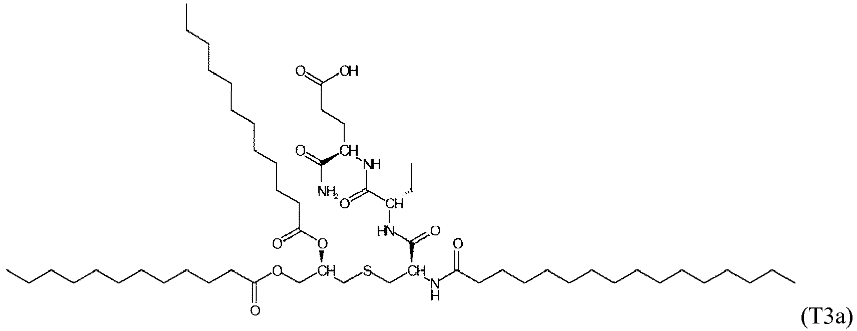

The agonist of formula T1 or T2 or T3a can be present as a pharmaceutically acceptable salt, a pharmaceutically acceptable solvate (*e.g*. hydrate), as a N-oxide derivative, as an isomer (including a tautomer or an enantiomer) or a mixture of isomers, *etc.* One particularly useful salt is the arginine salt of compound Tlc, which can be used as the arginine salt monohydrate.

Other useful TLR agonists are the following compounds:

| | |
|---|---|
| 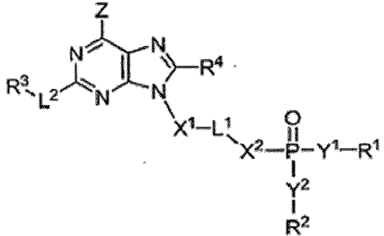 | 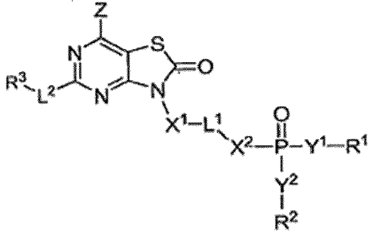 |
| as defined on pages 2-7 of WO2008/005555; | as defined on pages 2-5 & 7-8 of WO2008/005555; |
| 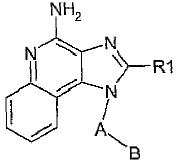 | 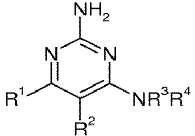 |
| as defined on pages 6 & 7 of WO2009/118296; | as defined on pages 2-5 ot WO2009/067081. |
| 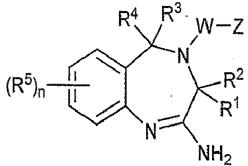 | 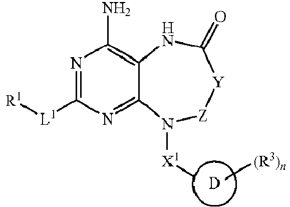 |
| as defined on pages 5-6 of WO2007/040840; | as defined on pages 2 to 3 of US2010/0143301. |
| 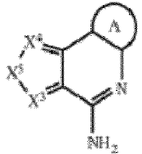 | |
| as defined on pages 2-4 of WO2009/111337 | |

Various useful TLR4 agonists are known in the art, many of which are analogs of endotoxin or lipopolysaccharide (LPS), or of monophosphoryl lipid A ('MPLA'). For instance, a TLR4 agonist used with the invention can be:
(i) 3d-MPL (*i.e.* 3-O-deacylated monophosphoryl lipid A; also known as 3-de-O-acylated monophosphoryl lipid A or 3-O-desacyl-4'-monophosphoryl lipid A). This derivative of the monophosphoryl lipid A portion of endotoxin has a de-acylated position 3 of the reducing end of glucosamine. It has been prepared from a heptoseless mutant of *Salmonella minnesota,* and is chemically similar to lipid A but lacks an acid-labile phosphoryl group and a base-labile acyl group. Preparation of 3d-MPL was originally described in GB-A-2220211, and the product has been manufactured and sold by Corixa Corporation. It is present in GSK's 'AS04' adjuvant. Further details can be found in Myers et al. (1990) pages 145-156 of Cellular and molecular aspects of endotoxin reactions*,* Johnson et al. (1999) J Med Chem 42:4640-9; Baldrick et al. (2002) Regulatory Toxicol Pharmacol 35:398-413)
(ii) glucopyranosyl lipid A (GLA) (Coler et al. (2011) PLoS ONE 6(1):e16333) or its ammonium salt *e.g.* 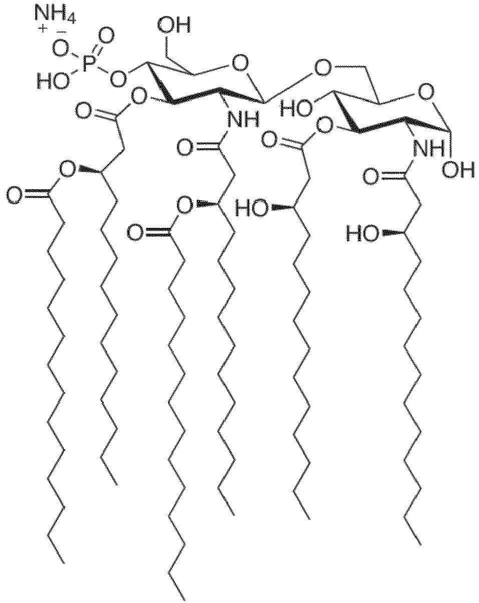
(iii) an aminoalkyl glucosaminide phosphate, such as RC-529 or CRX-524 (Johnson et al. (1999) Bioorg Med Chem Lett 9:2273-2278; Evans et al. (2003) Expert Rev Vaccines 2:219-229; Johnson et al. (1999) Bioorg Med Chem Lett 9:2273-2278; Evans et al. (2003) Expert Rev Vaccines 2:219-229; Bazin et al. (2006) Tetrahedron Lett 47:2087-92) . RC-529 and CRX-524 have the following structure, differing by their R₂ groups: 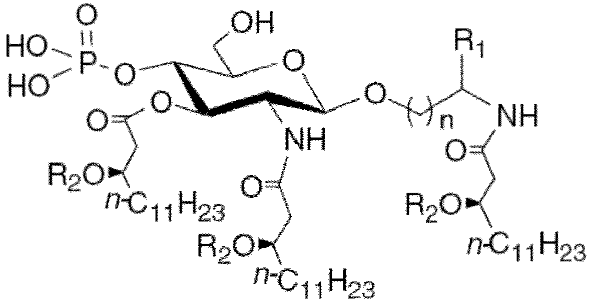
   R₁ = H, R₂= n-C₁₃H₂₇CO, n=1 (RC-529)
   R₁ = H, R₂= n-C₉H₁₉CO, n=1 (CRX-524)
(iv) compounds containing lipids linked to a phosphate-containing acyclic backbone, such as E5564 (Wong et al. (2003) J Clin Pharmacol 43(7):735-42 ; US2005/0215517.) 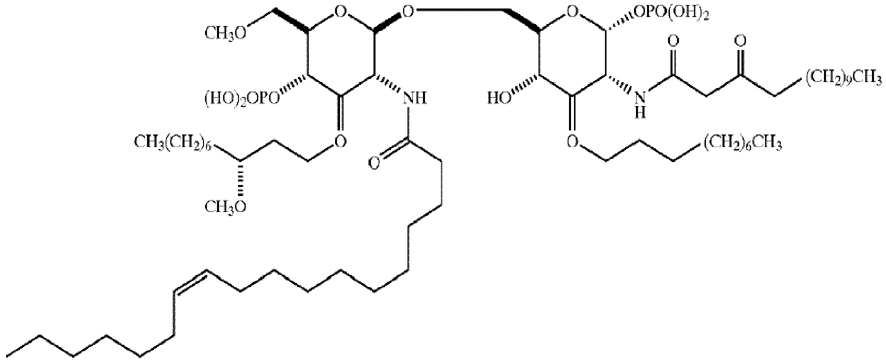
(v) A compound of formula I, II or III as defined in WO03/105769, or a salt thereof, such as compounds 'ER 803058', 'ER 803732', 'ER 804053', 'ER 804058', 'ER 804059', 'ER 804442', 'ER 804680', 'ER 803022', 'ER 804764' or 'ER 804057'. ER804057 is also known as E6020 and it has the following structure: 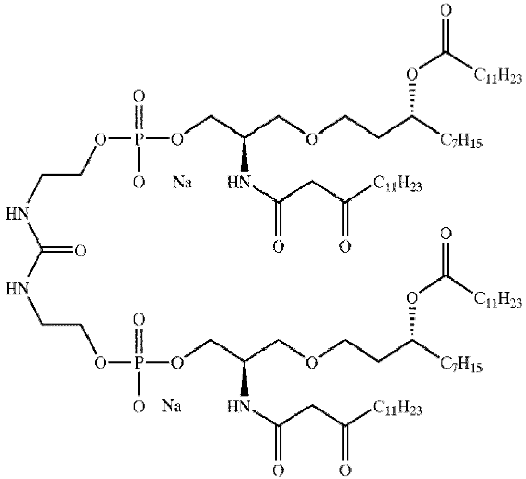 whereas ER 803022 has the following structure: 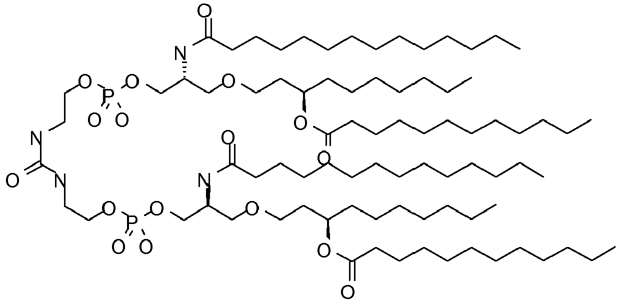
(vi) One of the polypeptide ligands disclosed in Peppoloni et al. (2003) Expert Rev Vaccines 2:285-93

Preferred TLR4 agonists are analogs of monophosphoryl lipid A (MPL)

Additional immunopotentiators could also include immunopotentiators (SMIPs) which do not act via TLRs. In particular, SMIPs which may be used with the invention may agonise C-type lectin receptors (CLRs) or CD1d rather than (or in addition to) a TLR. Thus the present disclosure includes the invention as described above with reference to TLR agonism, but wherein references to a TLR agonist (or similar) are replaced by reference either to a CLR agonist or to a CD1d agonist.

CLR agonists include, but are not limited to, trehalose-6,6'-dimycolate (TDM), its synthetic analog D-(+)-trehalose-6,6'-dibehenate (TDB), and other 6,6'-diesters of trehalose and fatty acids. Thus the invention can be applied to trehalose esters and diacyl trehaloses which are CLR agonists. These agonists may have formula (C): 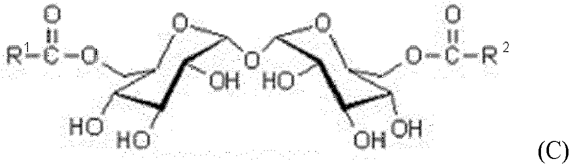 where R¹C(O)- and R²C(O)- are the same or different and are acyl groups. Suitable acyl groups may be saturated or unsaturated. They may be selected from the acyl residues of a mycolic acid, a corynomycolic acid, a 2-tetradecyl-3-hydroxyoctadecanoic acid, a 2-eicosyl-3-hydroxytetracosanoic acid, a bourgeanic acid, a behenic acid, a palmitic acid, *etc.* Useful mycolic acids include alpha-, methoxy-, and keto- mycolic acids, in cis- and or trans- forms.

CD1d agonists include, but are not limited to, α-glycosylceramides (De Libero et al, Nature Reviews Immunology, 2005, 5: 485-496; US patent 5,936,076; Oki et al, J. Clin. Investig., 113: 1631-1640 US2005/0192248; Yang et al, Angew. Chem. Int. Ed., 2004, 43: 3818-3822; WO2008/047249; WO2008/047174) such as α-galactosylceramides. Thus the invention can be applied to glycosylceramides which are CD1d agonists, including α-galactosylceramide (α-GalCer), phytosphingosine-containing α-glycosylceramides, [(2S,3S,4R)-1-O-(α-D-galactopyranosyl)-2-(N-hexacosanoylamino)-1,3,4-octadecanetriol], OCH, KRN7000 CRONY-101, 3"-O-sulfo-galactosylceramide, *etc.*

In some embodiments, the invention uses an 'Alum' adjuvant (*e.g*. in combination with a TLR agonist, as described above). The term 'Alum' refers herein to an aluminum salt, and useful aluminium salts include but are not limited to aluminium hydroxide and aluminium phosphate adjuvants. Such salts are described *e.g.* in chapters 8 & 9 of reference 54. Aluminium salts which include hydroxide ions are the preferred aluminium salts for use with the present invention *e.g.* aluminium hydroxides and/or aluminium phosphates (which includes aluminium hydroxyphosphates). An aluminium hydroxide adjuvant is most preferred. A composition can include a mixture of both an aluminium hydroxide and an aluminium phosphate. The concentration of Al⁺⁺⁺ in a composition for administration to a patient is preferably less than 1mg/ml, and a maximum of 0.85mg per unit dose is preferred.

### Packaging of vaccine compositions

Suitable containers for compositions of the invention (or kit components) include vials, syringes (*e.g.* disposable syringes), nasal sprays, *etc.* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g.* 10 doses. Preferred vials are made of colourless glass.

A vial can have a cap *(e.g.* a Luer lock) adapted such that a pre-filled syringe can be inserted into the cap, the contents of the syringe can be expelled into the vial (*e.g.* to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"™.

Containers may be marked to show a half-dose volume *e.g.* to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A kit or composition may be packaged (*e.g*. in the same box) with a leaflet including details of the vaccine *e.g.* instructions for administration, details of the antigens within the vaccine, *etc.* The instructions may also contain warnings *e.g*. to keep a solution of adrenaline readily available in case of anaphylactic reaction following vaccination, *etc.*

### Methods of treatment, and administration of the vaccine

In one aspect, the invention provides a method of administering an influenza vaccine to a patient who has been found to be negative for the HLA DQB 1*0602 haplotype, preferably a vaccine comprising at least one influenza A virus. In 2009, all patients which developed symptoms of narcolepsy following administration of the Pandemrix™ vaccine were found to have this phenotype and it is therefore desirable to exercise specific caution with this patient group.

The testing of the patient and the administration of the influenza vaccine may occur substantially at the same time (for example, during the same visit to a healthcare professional). It is more common, however, that the patient is tested some time before receiving the influenza vaccine. For example, the testing step and the administration step may be performed days, months or even years from each other.

Methods of testing a patient for the HLA DQB 1*0602 haplotype are known in the art. Such methods may involve, for example, the sequencing of the haplotype or the PCR amplification of at least part of the HLA. It can also be conducted using haplotype specific probes (for example, in southern blot assays) which can distinguish the different HLA haplotypes. The patient may be homozygous for HLA DQB 1*0602; or the patient may be heterozygous for the HLA DQB 1*0602 haplotype in which case it can still be advantageous to exclude such patients from receiving an influenza vaccine.

The invention provides a vaccine manufactured according to the invention. These vaccine compositions are suitable for administration to human or non-human animal subjects, such as pigs or birds, and the invention provides a method of raising an immune response in a subject, comprising the step of administering a composition of the invention to the subject. The invention also provides a composition of the invention for use as a medicament, and provides the use of a composition of the invention for the manufacture of a medicament for raising an immune response in a subject.

The immune response raised by these methods and uses will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [67]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (e.g. into the arm or leg), but other available routes include subcutaneous injection, intranasal [68-70], oral [71], intradermal [72,73], transcutaneous, transdermal [74], *etc.*

Vaccines prepared according to the invention may be used to treat both children and adults. Influenza vaccines are currently recommended for use in pediatric and adult immunisation, from the age of 6 months. Thus a human subject may be less than 1 year old, 1-5 years old, 5-15 years old, 15-55 years old, or at least 55 years old. Preferred subjects for receiving the vaccines are the elderly *(e.g.* ≥50 years old, ≥60 years old, and preferably ≥65 years), the young *(e.g.* ≤5 years old), hospitalised subjects, healthcare workers, armed service and military personnel, pregnant women, the chronically ill, immunodeficient subjects, subjects who have taken an antiviral compound (*e.g*. an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naïve patients *e.g*. for people who have never received an influenza vaccine before, or for vaccinating against a new HA subtype (as in a pandemic outbreak). Multiple doses will typically be administered at least 1 week apart (*e.g.* about 2 weeks, about 3 weeks, about 4 weeks, about 6 weeks, about 8 weeks, about 10 weeks, about 12 weeks, about 16 weeks, *etc*.).

Vaccines produced by the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (*e.g.* during the same medical consultation or visit to a healthcare professional) an antiviral compound, and in particular an antiviral compound active against influenza virus (e.g. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof *(e.g.* the ethyl esters) and salts thereof *(e.g.* the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU™).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x is optional and means, for example, *x*±10%.

The term "corresponding" in relation to two amino acids or amino acid sequences refers to amino acids which are aligned to each other when sequences are aligned by a pairwise alignment algorithm. The preferred pairwise alignment algorithm for use in identifying "corresponding" amino acid(s) is the Needleman-Wunsch global alignment algorithm [75], using default parameters (*e.g*. with Gap opening penalty = 10.0, and with Gap extension penalty = 0.5, using the EBLOSUM62 scoring matrix). This algorithm is conveniently implemented in the *needle* tool in the EMBOSS package [76]. The same principle applies to the term "equivalent" as used herein in relation to sequence comparisons *i.*e to determine whether a given sequence has a sequence equivalent to amino acids 106 to 126 of SEQ ID NO: 1 or 2 would typically involve an alignment as described above.

Unless specifically stated, a process comprising a step of mixing two or more components does not require any specific order of mixing. Thus components can be mixed in any order. Where there are three components then two components can be combined with each other, and then the combination may be combined with the third component, *etc.*

The various steps of the methods may be carried out at the same or different times, in the same or different geographical locations, *e.g*. countries, and by the same or different people or entities.

Where animal (and particularly bovine) materials are used in the culture of cells, they should be obtained from sources that are free from transmissible spongiform encephalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 77. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is taught in reference 78.

References to a percentage sequence identity between two nucleic acid sequences mean that, when aligned, that percentage of bases are the same in comparing the two sequences. This alignment and the percent homology or sequence identity can be determined using software programs known in the art, for example those described in section 7.7.18 of reference 77. A preferred alignment program is GCG Gap (Genetics Computer Group, Wisconsin, Suite Version 10.1), preferably using default parameters, which are as follows: open gap = 3; extend gap = 1.

### DESCRIPTION OF THE DRAWINGS

**Figure 1** Smith-Waterman alignments between the nucleocapsid X-179A/X-181 sequence fragments from Table 3 and orexin receptor 2 (Ox1R) and orexin receptor 1 (Ox2R). No other alignments between influenza fragments and orexin family sequences were less than 0.4. The alignments were generated with the program search from the FASTA package with default parameters. The regions of Ox2R and Ox1R in the alignments shown in are annotated as extracellular in Uniprot.

### EXAMPLES

### Narcolepsy and the H1N1 Pandemic

Molecular mimicry is an evolutionary adaptation whereby viruses and bacteria attempt to fool the body into granting them free access to the host tissue. Such mimicry works by showing the immune system stretches of amino acids that look like self. In responding to the microbe, the immune system becomes primed to attack the corresponding self-component (*e.g*. adenovirus type 2 and myelin basic protein in multiple sclerosis).

### Autoimmune Diseases and Natural Infections

What is commonly observed by clinicians managing patients with autoimmune diseases is that natural infection can trigger and augment the severity of autoimmune disease activity. Similarly, natural infections are thought to play a major role in inducing disease in a genetically susceptible host. In the context of the H1N1 pandemic, 153 subjects were infected with H1N1 in Beijing, China, by May 2009 which increased to an estimated 1.18 million infected subjects by November 2009. By November, 1.36 million doses of unadjuvanted H1N1 vaccine had been administered to a population of 17 million (*i.e.* 0.8% of the population were vaccinated). Six months following the peak of H1N1 infection, there was a report of a 3 to 4-fold increase in new narcolepsy cases (n=142) in a Beijing cohort in which only 5.6% patients reported being vaccinated. This suggested to the inventors that the narcolepsy seen in Pandemrix™-treated patients might be connected to the H1N1 strain itself, perhaps due to molecular mimicry.

### Pathological Mimicry Related to the Pandemrix™ Vaccine Antigen

As mentioned above, the Pandemrix™ vaccine was associated with narcolepsy while no increase in narcolepsy could be seen following vaccination with Focetria™. The source of the vaccine antigens used for Pandemrix™ is the high yielding A H1N1 reassortant X-179A while that used for Focetria™ is the higher-yielding A H1N1 reassortant X-181.

For pandemic H1N1 vaccine preparation, X-179A (used for Pandemrix™) was generated by the cross of high yielding strain X-157, with internal proteins traceable to A/Puerto Rico/8/1934 (PR8), with A/California/07/2009 (H1N1 subtype) which contributed the surface antigens HA and NA, and the internal protein PB1. Due to concerns of inadequate yield, the 32-fold yielding X-179A was re-reassorted by crossing again to X-157 on July 14, 2009 leading to the generation of the 64-fold yielding X-181 (used in Focetria™ and other seasonal vaccines). This functional difference is related not only to the gene segment combinations from the reassortment, but also pre-existing variants and virus mutations selected during adaptation to the egg host (*in ovo* inoculation). A similar result occurred with X-53 and X-53a reassortants prepared for the swine flu vaccine in 1976 which were not antigenically distinguishable but had a single amino acid change in the HA gene of X-53a that increased the yield by 16-fold. Thus, X-179A used for Pandemrix™ is likely to have other qualitative differences compared to X-181 used for Focetria™ as will be explained below.

The purification processes for Pandemrix™ and Focetria™ have distinct differences. Split-virion vaccines (like Pandemrix™) and subunit vaccines (like Focetria™) are defined in the World Health Organization document on production technologies for influenza vaccines as follows:
The majority of influenca vaccines are split' vaccines, which are produced by *The majority of influenza vaccines are 'split' vaccines, which are produced by detergent-treating purified influenza virus. The splitting process breaks the virus allowing the relevant antigens to be partially purified (p.8)...Compared to the whole-virus preparation, split vaccines are better characterized, contain less ovalbumin and are claimed to be less reactogenic (p.13). Subunit or surface surface-antigen vaccines are produced as for split virus, but more rigorous purification is carried out so that the vaccine consists almost exclusively of highly purified HA and NA with minimal contaminating N, matrix protein, nucleoprotein and lipid.*

The amount of nucleoprotein and matrix protein content in an intact influenza virus is two-fold and six-fold higher, respectively, to that of HA making these two internal proteins most likely to carry over depending on the type of purification process used to enrich for HA and NA. Indeed, a previous investigation characterized the split-virion vaccines Fluzone and MFV-Ject and the purified antigen/subunit influenza vaccines Fluvirin and Influvac that were available in the United Kingdom in 1992 [79]. Based on electron microscopy, viral nucleoprotein is readily evident in split-virion vaccines and by SDS-PAGE gel is detectable in all vaccines to varying levels (split-virion > purified antigen/subunit).

Since mutations of orexin receptors and loss of orexin cells have been implicated in narcolepsy, we have hypothesized that a vaccine antigen in Pandemrix™ could have a unique characteristic leading to molecular mimicry with either orexin (hypocretin) or its receptors. Therefore, sequence analysis was performed on the influenza proteins from X-179A, X-181 and orexin-related sequences (orexin, orexin A, orexin B, orexin receptor 1, orexin receptor 2 and HLA-DQB1). As the Pandemrix™ strain X-179A is implicated in the narcolepsy cases but Focetria™ is not, the influenza proteins were first compared for differences between the vaccine viral strains X-179A (Pandremix-associated) and X-181 (Focetria™-associated) that could account for the association with narcolepsy. Only proteins expected to be present in the vaccine preparations were included (HA, NA, NP and M1) as suggested by the electron microscopy and SDS-PAGE studies previously described.

To determine potential amino acid changes, influenza sequences were retrieved from the NCBI's Influenza Virus Resource [80] querying by the appropriate strain names (X-179A, X-181, A/California/07/2009(H1N1)) with duplicate sequences removed. Each influenza protein expected to be present in the vaccine preparations (HA, NP, M1, M2, NA) was compared across strains to identify sequences where X-179A differed from X-181 by at least one residue. Three amino acid differences were found with one in HA and two in NP (Table 1):

| **Protein** | **Genbank Accession** | **Strain** | **Sequence** | **SEQ ID NO:** |
|---|---|---|---|---|
| HA | ACR47014 | X-179A | 136 KTSSWPNHDS**N**KGVTAACPHA | 6 |
| | AFM72842 | X-181 | 136 KTSSWPNHDS**D**KGVTAACPHA | 7 |
| NP | ADE29096 | X-179A | 106 RELILYDKEE**I**RRIWRQANNG | 1 |
| | AFM72846 | X-181 | 106 RELILYDKEE**M**RRIWRQANNG | 3 |
| | ADE2096 | X-179A | 130 WRQANNGDD**A**AAGLTHMMIWH | 4 |
| | AFM72846 | X-181 | 130 WRQANNGDDA**T**AGLTHMMIWH | 5 |

Sub-sequences including ten residues before and after the differences were then compared to the orexin related sequences using a Smith-Waterman alignment. Only four alignments had an e-value below 0.4 (the next best e-value is >0.4 giving a ratio of the best e-value alignment to the next best of 10:1 which is a good separation of the indicated alignment from the others). The alignments were between a nucleocapsid protein fragment from X-179A and X-181 (containing a single residue difference) and the orexin receptors 1 and 2 (Figure 1). As the X-179A version of the nucleocapsid fragment overlaps with numerous epitopes reported in the Immune Epitope Database (www.iedb.org), this overlap with orexin receptors 1 and 2 (which are implicated in narcolepsy) suggests the potential for mimicry and is a possible explanation for the observations of narcolepsy in certain European countries following administration of the Pandemrix™ vaccine. Interestingly, the X-179A version of the nucleocapsid fragment is also identical to that from the H1N1 infection (consistent with the reported association of infection with narcolepsy [81]) while the X-181 version (Focetria™-linked) of the same nucleocapsid fragment contains a single amino acid substitution that could explain the lack of narcolepsy association with Focetria™.

Patients with narcolepsy associated with Pandemrix™ are exclusively HLA DQB1*0602. Thus in Sweden all 28 post-vaccination cases of narcolepsy were HLA DQB1*0602. In Finland, all 34 HLA typed narcolepsy patients in 2010 who were vaccinated with Pandemrix™ were HLA DQB1 *0602. The binding motif for HLA DQB 1*0602 is known. The core binding motifs for HLA DQB 1*0602 are at positions 1, 3, 4, 6, and 9. There is a register for the orexin receptor 1 and 2 peptides that fits this motif well for the peptide **L**I**LY**DKEE**I**RRIWRQANNG (SEQ ID NO: 10) with aliphatic amino acids at position 1 and 3, and a hydrophobic amino acid at position 4. Though position 6 does not fit the motif, position 9 is aliphatic and provides a good fit [82]. In narcolepsy the P4 binding pocket with the largest volume is critical for susceptibility, and some known examples of strong binders to 0602 have tyrosine at P4 [8].

The Pandemrix™ vaccine may further have included certain components of the H1N1 infectious agent responsible for immune responses to molecular mimics of self-antigens (hypocretin or one of its receptors) leading to narcolepsy. However, one must still exercise appropriate vigilance before drawing definitive conclusions because of the discordance in narcolepsy signals associated with the AS03-adjuvanted Pandemrix™ vaccine and the AS03-adjuvanted H1N1 pandemic vaccine Arepanrix (administered in Canada with no report of increased narcolepsy associated with vaccination). This might suggest that simply the presence of a pathogenic antigen may alone not be enough to explain the association or, alternatively, there may be differences in the presence or presentation of the split vaccine antigens in the AS03-adjuvanted vaccines due to differences in the splitting/purification process of the manufacturing sites (Dresden for Pandemrix™ and Quebec for Canada). An estimated 30.8 million doses of the GSK AS03-adjuvanted H1N1 vaccine manufactured in Dresden were used in more than 47 countries starting in October 2009 with high coverage in some countries including Finland, Sweden, Norway, and Ireland. The GSK AS03-adjvuanted H1N1 vaccine manufactured in Quebec was used with high coverage in Canada (Arepanrix) where an estimated 12 million doses were administered and also administered in several other countries. Epidemiological studies that are on-going in Canada will report on any association between narcolepsy and the AS03-adjuvanted H1N1 vaccine (Arepanrix) in due course.

The Dresden antigen contains Polysorbate 80 (Tween 80) and Triton X-100, while the Quebec antigen does not contain these excipients. It has been speculated that these detergents might have an effect on the development of Narcolepsy (see Assessment report Immunological differences between pandemic vaccines EMA/687578/2012). Therefore it might be of particular importance to avoid the presence of NP protein in vaccines produced from antigens which contain Tween 80 and/or Triton X-100 like in the Dresden antigen. This applies in particular to vaccines adjuvanted with oil-in-water emulsions like MF59 or AS03, as narcolepsy has not appeared in unadjuvanted seasonal vaccines derived from the Dresden antigen (see below).

The following table 2 shows the composition of 2 different inactivated split virion H1N1 antigen components prepared in Dresden (WO2011/051235 p 41).

| **Ingredient** | **Quantity per 0.25 ml - DFLSA013A (initial process)** | **Quantity per 0.25 ml - DFLS014A (adapted process)** | **Unit** |
|---|---|---|---|
| Purified antigen fractions of inactivated split virion A/California/7/2009 (H1N1)v NYMC X-179A | 3.75 | 3.75 | µg HA |
| Polyoxyethylene sorbitan monooleate (TWEEN-80™, or polysorbate 80) | >28.75 | ≥28.75 | µg |
| t-octylphenoxypolyoxyethanol (TRITON X-100™) | 3.75 | 22.5 | µg |
| Sodium chloride | 1.92 | 1.92 | mg |
| Disodium phosphate | 0.26 | 0.26 | mg |
| Potassium dihydrogen phosphate | 0.094 | 0.094 | mg |
| Potassium chloride | 0.050 | 0.050 | mg |
| Magnesium chloride | 0.012 | 0.012 | mg |
| Thiomersal | 5 | 5 | µg |
| Water for injections q.s. ad. | 0.25 | 0.25 | ml |

### Absence of Narcolepsy in the GSK Non-Adjuvanted H1N1 Seasonal Influenza Vaccine

While both the pandemic and seasonal influenza vaccines manufactured by GSK contain the same H1N1 antigen, there is no narcolepsy signal reported with the non-adjuvanted H1N1 antigen in the seasonal vaccine that could immediately lead one to speculate that excessive immunostimulation by the adjuvant is causal for narcolepsy. However, in light of the previous discussion on cryptic antigens being revealed in the split-virus vaccines, one could as well explain the discordant narcolepsy association to the reservoir of pathological antigens being preferentially or more efficiently presented to the immune system by the AS03 adjuvant - keeping in mind that improved antigen presentation is a desirable and expected effect with any adjuvants. Adjuvants may act in several ways including the following: 1) delivering antigens to the immune system, 2) enhancing the uptake of the antigen by antigen-presenting cells (APCs), or 3) altering the structural conformation of the antigen within the vaccine, thereby allowing for progressive release, delayed clearance and better exposure to the immune system. The mode of action of oil-in-water emulsions is being better understood and currently is considered to involve modulation of innate inflammatory responses, APC recruitment and activation, enhancement of antigen persistence at the injection site, modulation of presentation of antigen to immune-competent cells, and elicitation of different patterns of cytokines.

### Immunological Explanation

Pertinent findings from a conservancy analyses published in 2007 on antibody and T cell epitopes of influenza A virus using the Immune Epitope Database and Analysis Resources (IEDB) are the paucity of antibody epitopes in comparison to T-cell epitopes with the highest number of T-cell epitopes being derived from hemagglutinin protein and nucleoprotein [83]. Furthermore, T-cell epitopes are more conserved than antibody epitopes with 50% being conserved at 80% identity levels in human H1N1 strains suggesting significant levels of interstrain cross-reactivity for T-cell epitopes in influenza. Nucleoprotein of influenza A is efficiently presented by class I and class II major histocompatibility complexes and is capable of expanding both CD8+ and CD4+-specific effector T lymphocytes secreting gamma-interferon and tumor necrosis factor [84]. It is a major target antigen for cross-reactive anti-influenza A cytotoxic lymphocytes (CTL), and recombinant vaccinia virus containing the PR8 nucleoprotein gene can both stimulate and prime for a vigorous secondary cross-reactive CTL response [85]. It is precisely for this reason why split-virion influenza vaccines that contain significant quantities of non-surface proteins would be expected to increase cell-mediated immune responses compared to purer subunit vaccines. However, this is a double-edged sword because the same immunological mechanism generating a vigorous cross-reactive CTL response to a defined epitope of viral (or vaccine-modified) nucleoprotein can be problematic if it mimics host tissue (*e.g*., orexin receptors) leading to T-cell-mediated autoimmunity that degenerates in a genetically susceptible host (*e.g.,* DBQ1*0602) into autoimmune disease (*e.g.,* narcolepsy). The alignment of the Pandemrix™ nucleoprotein fragment with the orexin receptors is intriguing because distinct narcolepsy syndromes have also been generated in orexin 2 receptor knockout mice and orexin knockout mice (possibly through defective orexin to orexin 1 receptor signaling) [86]. Interestingly, if trace amounts of immunogenic nucleoprotein were present in Focetria™, there is the presence of one amino-acid substitution (methionine) in the nucleoprotein fragment aligning with orexin receptors that distinguishes it from that of Pandemrix™ nucleoprotein and H1N1 infection (both of which have isoleucine). This amino acid substitution in nucleoprotein contained in Focetria™ (inherited from the X-181 vaccine strain) may be functionally similar to that for influenza viruses in which a number of amino acid substitutions in the nucleoprotein enable escape from CTL-mediated immune surveillance in contrast to matrix protein that is highly conserved [87].

### HLA haplotype binding assays

Binding of peptides to various HLA-DRB1* haplotypes was analyzed by using the cell-free REVEAL™ class II binding technology (see ref. 88). This technology measures the ability of synthetic test peptides to stabilize MHC-peptide complexes. Detection is based on the presence or absence of the native conformation of the MHC-peptide complex, which is detected by a specific monoclonal antibody. Each peptide is given a score relative to a positive control peptide, which is a known T-cell epitope. The score is reported quantitatively as a percentage of the signal generated by the test peptide compared with the positive control peptide. Scores are assessed at time zero and again after 24 hours. The analysis also gives a stability index which represents the stability of the binding of each peptide with the MHC II complex being tested.

A total of 18 peptides were tested, plus a positive control:

| **Peptide** # | **Sequence** | **SEQ ID NO:** | **Details** |
|---|---|---|---|
| 1 | RELILYDKEEIRRIWRQANNG | 1 | X179-A NP fragment |
| 2 | RELILYDKEEMRRIWRQANNG | 3 | X181 NP fragment |
| 3 | LILYDKEEIRRIWRQ | 18 | X179-A NP fragment |
| 4 | LILYDKEEMRRIWRQ | 19 | X181 NP fragment |
| 5 | VGKMIGGIGRFYIQM | 20 | Common NP sequence |
| 6 | SGAAGAAVKGVGTMV | 21 | Common NP sequence |
| 7 | EKATNPIVPSFDMSN | 22 | Common NP sequence |
| 8 | IDPFKLLQNSQVVSL | 23 | Common NP sequence |
| 9 | LILYDKEERRRRWRQ | 24 | Mutant of #3 |
| 10 | MNLPSTKVSWAAVTL | 25 | Orexin DQB1*0602 fragment |
| 11 | MNLPSIKVSWAAVTL | 26 | Mutant of #10, Thr→Ile |
| 12 | MNLPSMKVSWAAVTL | 27 | Mutant of #10, Thr→Met |
| 13 | LTVAAWSVKTSPLNM | 28 | Reverse of #10 |
| 14 | GAGNHAAGILTLGKR | 29 | Orexin fragment HCRT56-68 |
| 15 | ASGNHAAGILTMGRR | 30 | Orexin fragment HCRT87-99 |
| 16 | AMERNAGSGIIISDT | 31 | Hemagglutinin fragment |
| 17 | ALNRGSGSGIITSDA | 32 | Hemagglutinin fragment |
| 18 | ALSRGFGSGIITSNA | 33 | Hemagglutinin fragment |

Two HLA haplotypes were tested: DQA1*0102:DQB1*0602; and DQA1*0101:DQB1*0501. As discussed above, the DQB1*0602 haplotype has a known link to narcolepsy, but the DQB1*0501 haplotype seems to protect against development of narcolepsy based on HLA typing studies of patients.

Binding results were as follows:

| | **DQB1*0501** | | | **DQB1*0602** | | |
|---|---|---|---|---|---|---|
| **Peptide** # | **REVEAL 0 hrs** | **REVEAL 24 hours** | **Stability index** | **REVEAL 0 hrs** | **REVEAL 24 hours** | **Stability index** |
| 1 | 27.8 | 7.4 | 3.5 | 24.4 | 16.9 | 11.1 |
| 2 | 1.1 | 1.0 | 1.1 | 1.5 | 0.5 | 0.2 |
| 3 | 4.7 | 1.2 | 0.6 | 1.2 | 0.4 | 0.2 |
| 4 | 0.1 | 0.1 | 0.1 | 0.5 | 0.4 | 0.6 |
| 5 | 43.6 | 13.9 | 6.4 | 77.3 | 52.3 | 33.0 |
| 6 | 0.0 | 0.0 | 0.0 | 0.1 | 0.1 | 0.1 |
| 7 | 0.0 | 0.0 | 0.0 | 0.0 | 0.0 | 0.0 |
| 8 | 0.0 | 0.0 | 0.0 | 0.1 | 0.1 | 0.1 |
| 9 | 19.0 | 6.4 | 2.9 | 22.3 | 17.3 | 14.7 |
| 10 | 0.2 | 0.1 | 0.1 | 1.7 | 0.3 | 0.2 |
| 11 | 18.0 | 2.1 | 1.4 | 47.5 | 20.8 | 9.6 |
| 12 | 2.5 | 0.8 | 0.4 | 12.3 | 3.7 | 1.7 |
| 13 | 0.3 | 0.3 | 0.4 | 0.9 | 0.4 | 0.2 |
| 14 | 0.0 | 0.0 | 0.0 | 0.1 | 0.1 | 0.1 |
| 15 | 0.1 | 0.1 | 0.1 | 0.2 | 0.2 | 0.3 |
| 16 | 0.1 | 0.1 | 0.1 | 0.0 | 0.0 | 0.0 |
| 17 | 0.0 | 0.0 | 0.0 | 0.0 | 0.0 | 0.0 |
| 18 | 0.0 | 0.0 | 0.0 | 0.0 | 0.0 | 0.0 |
| Ctrl | 100.0 | 14.5 | 8.7 | 100.0 | 67.4 | 42.5 |

There were four strong binders for the DQB1*0602 HLA haplotype, namely peptides #1, #5, #9 and #11 (>15% of the positive control signal). In contrast to the X179-A peptide (#1) which was strongly bound at time 0 and still 24 hours later, the corresponding fragment from X181 (#2) was a poor binder at both time points.

In general, the binding stability of all peptides is weaker with the DQB1*0501 haplotype. Peptide #1 still binds well but is clearly less stable after 24 hours. Peptide #3 (a shorter version of peptide #1) binds better to this haplotype than to DQB 1*0602, whereas #4 (a shorter version of #2) remains a poor binder with the 0501 haplotype. Thus the, compared to DQB1*0602, which is clearly associated with narcolepsy patients, the NP peptides bound poorly to the haplotype which seems to protect against development of narcolepsy (DQB1*0501).

A 15-mer orexin fragment (#10) did not bind to either HLA haplotype, but a modified version of this peptide with a Thr→Ile mutation (#11) was a strong binder. The result for peptide #10 is not surprising in view of reference 8's report that the fragment needed a 15 aa linker in order to "facilitate complex formation" with the DQB1*06:02 HLA. The ability of the Thr→Ile mutation to convert the peptide into a strong binder supports the importance of the isoleucine in NP of strain X179-A in conferring affinity for the DQB1*06:02 HLA haplotype.

The result with peptide #12 demonstrates that changing the threonine in peptide #10 to methionine does not allow or improve binding to HLA DQB 1*0602 (the allele associated with narcolepsy) and thus confirms the result seen with peptides #1 and #2 regarding DQB 1*0602

Overall, these results show that peptides #1 and #2 exhibit markedly different binding to HLA-DQA1^{*}01:02 DQB1*06:02. Peptide #1 exhibits good binding to this allele, and its high stability score suggests that the binding is strong. In contrast, peptide #2 exhibits low initial binding, and only a very small amount of complex remains after 24 hours, suggesting a short binding half-life, and an unstable complex. Thus peptide #1 (amino acids 106-126 of the X-179A nucleoprotein and containing an isoleucine residue at amino acid position 116) bound more strongly and with better stability than peptide #2 (amino acids 106-126 of the X-181 nucleoprotein and containing a methionine residue at amino acid position 116). Accordingly, these data support our hypothesis that the X-179A nucleoprotein, but not the X-181 nucleoprotein, may be involved in an autoimmune response specific to individuals with the HLA-DQB 1*06:02 haplotype.

### Modeling of peptide interactions with orexin

The orexin (hypocretin) fragment 1-13 (SEQ ID NO: 16) is known to interact strongly with HLA DQB1*0602 [8]. Analysis shows that Leu-3, Thr-6 and Val-8 are key residues for the interaction. These three residues give a good 3D structural alignment with a 12-mer fragment of the X-179A nucleoprotein fragment (SEQ ID NO: 17) with the influenza NP peptide in the reverse orientation, with Ile-6 aligning with orexin Thr-6 and Ile-9 aligning with Leu-3. The NP Ile-6 is the residue which differs between SEQ ID NOs: 1 (X-179A) and 3 (X-181). Computer modelling shows that SEQ ID NO: 17 shows a very good fit in the binding groove of the DQ0602 crystal structure. The Ile-6 residue in X-179A fits well in the HLA protein's binding cavity for orexin's Thr-6, but the X-181A methionine residue in the corresponding 12-mer fragment of SEQ ID NO: 3 gives a severe spatial clash with the HLA protein.

For this modeling the PDB file luvq was used with PyMol (Schrodinger Inc). An analysis of the intermolecular interactions between the hypocretin peptide (SEQ ID NO: 16) and the HLA protein was performed in detail, both visually and through the use of the SiteMap software (Schrodinger Inc). Three residues from the X-ray structure of the peptide were identified as important to drive binding to the HLA protein, most likely through hydrophobic interactions (Leu-3, Thr-6 and Val-8).

An alignment of a fragment of the nucleoprotein (SEQ ID NO: 17) was carried out and it showed many clashes and unlikely binding poses, until it was docked in the reverse orientation. A putative binding mode was evaluated to the binding site of HLA-DQB 1*0602 in terms of polarity and van der Waals clashes, and a good match was seen when Ile-6 was aligning with orexin Thr-6 and NP Ile-9 aligns with Leu-3. To avoid incorporating noise or bias into the alignment assessment, the following was done: (i) the orexin peptide was mutated into the NP peptide residue-by-residue using the mutation module of PyMol; (ii) at each position, the best conformers for each residue of the NP peptide were assessed to fit or not into the binding pocket by its proximity to the van der Waals surface of the HLA protein. The surface was generated with PyMol using the default normal external surface; (iii) the models generated for the NP peptide should fit as close as possible the location of the orexin template. A satisfactory model was generated by this method. One polarity mismatch originating in the alignment (NP Glu-4 vs. orexin Val-8) was further evaluated, but the Glu residue is spatially tolerated in the binding groove of the HLA protein (no clashes with the surface of the protein are observed).

To further analyse this binding hypothesis, the Ile/Met mutation (SEQ ID NOs: 1 and 3) was evaluated in the binding pocket following the same principles detailed above. When the Ile residue is mutated to Met, no conformer of Met could be found that did not severely clash with the surface of the HLA protein. From this analysis, it is concluded that the Met mutation cannot be tolerated in this pocket within the proposed structural alignment.

In parallel, both sequences (*i.e.* the Ile and Met variants) were modelled in DQB1*0302 (PDB 1jk8) and the pocket which would accommodate this residue (and which fits a Tyr residue from insulin in the published sequence) is flexible and so should not discriminate between Ile and Met.

Similarly, both NP sequences and the orexin fragment were modelled in a HLA-DQ2 structure (PDB 1s9v). The orexin peptide fits into the DQ2 groove with a loose fit and no severe clashes. In contrast, both NP peptides have a very severe clash protruding through the DQ2 surface, and this clash could not be cured in any rotamer.

Thus, these modelling studies are consistent with the MHC peptide binding study: of the three HLA molecules modelled, only DQB 1*0602 can discriminate between the NP peptides which differ by the Ile/Met variation.

Accordingly, both the MHC peptide binding study and the modelling study point to differential binding of certain X-179A versus X-181 nucleoprotein-derived peptides to DQB 1*0602 but not other HLA sub-types.

In addition, this sort of *in silico* modelling can be used to identify amino acid substitutions within the NP which should avoid strong binding to the DQB 1*0602 haplotype.

### Mass spectrometry analysis of influenza vaccines

Mass spectrometry was used to identify and quantify NP within five inactivated split influenza vaccines which include HA from the X-179A strain.

100µl samples of vaccines were acidified by addition of 10µL 50% formic acid water. Vaccines were vortexed and sonicated in a sonicating water bath for 10 minutes. Protein precipitation was performed with the addition of 500µL -80°C acetone and stored at -80°C for 2hrs. Samples were centrifuged at 4°C, 10,000rpm for 15 minutes. The supernatant was discarded and the protein pellet was dried for 10 minutes in a speed vac. Vaccines were reconstituted in 20µL 8M urea, 50mM ammonium bicarbonate and 20µL 0.5% protease, 50mM ammonium bicarbonate. Reduction was performed using DTT to a final concentration of 5mM, reduced at 55°C for 30 minutes. The samples were brought to room temperature and alkylated using propionamide at a final concentration of 100mM, room temperature for 30 minutes. 60µL of 50mM ammonium bicarbonate was added to each sample followed by 300ng of trypsin/LysC mix. The samples were digested overnight at 37°C, followed by acidification by the addition of 10µL 10% formic acid in water. The peptides were purified on C18 microspin columns and dried to less than 3µL in a speed vac.

Each sample was then reconstituted for HPLC-MSMS in 15µL 0.2% formic acid, 2% acetonitrile 97.8% water and injected onto a self-packed fused silica 25cm C18 reversed phase column. The flow rate was 300nL/minute with a linear gradient from 8% mobile phase B to 50% mobile phase B over 90minutes. The mass spectrometer was an LTQ Orbitrap Velos, set in data dependent acquisition (DDA) mode to fragment the 15 most intense multiply charged precursor ions, where these ions were placed on an exclusion list for 60 seconds. Results were searched on a sequence database using Byonic™ with tolerance settings of 10ppm on the precursor ion and 0.25Da on the fragment ions. A 1% FDR using a reverse decoy database approach was employed. The database contained all vaccine related protein sequences (310,490) from NCBI. For each vaccine the proteins were sorted by spectral count and a relative abundance calculation was made by summing the intensities of the top 10 proteins identified by spectral counts in each vaccine. The reported intensity for each of the top 10 proteins was divided by the summed intensity for these 10, multiplied by 100 and presented as a percentage top 10 abundance.

Spectral counts and percentage abundance values for NP and HA from the X-179 strain were:

| **Vaccine** | | **NP count** | **% abundance** | | **HA count** | **% abundance** |
|---|---|---|---|---|---|---|
| Fluzone™ 3-valent | | 452 | 13.9 | | 219 | 8.4 |
| Fluzone™ 4-valent | | 444 | 11.2 | | 252 | 5.7 |
| Fluarix™ 3-valent | | 517 | 18.4 | | 336 | 6.1 |
| Fluarix™ 4-valent | | 417 | 7.6 | | 196 | 3.9 |
| Afluria™ 3-valent | | 881 | 20.0 | | 118 | 4.0 |

In further analysis, peptides identified by MS were precisely matched by sequence to the strains known to be present in the vaccines, rather than by homology. Using this method the results were as follows:

| **Vaccine** | **NP count** | **HA count** | **NP:HA** |
|---|---|---|---|
| Fluzone™ 3-valent | 328 | 258 | 1.27 |
| Fluzone™ 4-valent | 320 | 335 | 0.96 |
| Fluarix™ 3-valent | 146 | 282 | 0.52 |
| Fluarix™ 4-valent | 142 | 187 | 0.76 |
| Afluria™ 3-valent | 212 | 604 | 0.35 |

It will be understood that the invention has been described by way of example only and modifications may be made whilst remaining within the scope and spirit of the invention.

### SEQUENCES

***SEQ ID NO: 1X-179A NP fragment 106-126)***
   RELILYDKEEIRRIWRQANNG
***SEQ ID NO: 2 (X-179A NP.full length; 498aa)*** 
1 MASQGTKRSY EQMETDGERQ NATEIRASVG KMIGGIGRFY IQMCTELKLS DYEGRLIQNS
61 LTIERMVLSA FDERRNKYLE EHPSAGKDPK KTGGPIYRRV NGKWMRELIL YDKEEIRRIW
121 RQANNGDDAA AGLTHMMIWH SNLNDATYQR TRALVRTGMD PRMCSLMQGS TLPRRSGAAG
181 AAVKGVGTMV MELVRMIKRG INDRNFWRGE NGRKTRIAYE RMCNILKGKF QTAAQKAMMD
241 QVRESRNPGN AEFEDLTFLA RSALILRGSV AHKSCLPACV YGPAVASGYD FEREGYSLVG
301 IDPFRLLQNS QVYSLIRPNE NPAHKSQLVW MACHSAAFED LRVLSFIKGT KVLPRGKLST
361 RGVQIASNEN METMESSTLE LRSRYWAIRT RSGGNTNQQR ASAGQISIQP TFSVQRNLPF
421 DRTTIMAAFN GNTEGRTSDM RTEIIRMMES ARPEDVSFQG RGVFELSDEK AASPIVPSFD
481 MSNEGSYFFG DNAEEYDN
***SEQ ID NO: 3 (X-181 NP fragment 106-126)***
   RELILYDKEEMRRIWRQANNG
***SEQ ID NO: 4 (X-179A NP fragment 130-150)***
   WRQANNGDDAAAGLTHMMIWH
***SEQ ID NO: 5 (X-181 NP fragment 130-150)***
   WRQANNGDDATAGLTHMMIWH
***SEQ ID NO: 6 (X-179A HA fragment 136-157)***
   KTSSWPNHDSNKGVTAACPHA
***SEQ ID NO: 7 (X-181 HA fragment 136-158)***
   KTSSWPNHDSDKGVTAACPHA
***SEQ ID NO: 8 (X-179A NP fragment 108-116)***
   LILYDKEEI
***SEQ ID NO: 9***
   LXLYXXXIXXXXXX
***SEQ ID NO: 10 (X-179A NP fragment 108-126)***
   LILYDKEEIRRIWRQANNG
***SEQ ID NO: 11***
   LILYDKEEX
***SEQ ID NO: 12 (X-181 NP full length; 498aa)*** 
1 MASQGTKRSY EQMETDGERQ NATEIRASVG KMIGGIGRFY IQMCTELKLS DYEGRLIQNS
61 LTIERMVLSA FDERRNKYLE EHPSAGKDPK KTGGPIYRRV NGKWMRELIL YDKEEMRRIW
121 RQANNGDDAT AGLTHMMIWH SNLNDATYQR TRALVRTGMD PRMCSLMQGS TLPRRSGAAG
181 AAVKGVGTMV MELVRMIKRG INDRNFWRGE NGRKTRIAYE RMCNILKGKF QTAAQKAMMD
241 QVRESRNPGN AEFEDLTFLA RSALILRGSV AHKSCLPACV YGPAVASGYD FEREGYSLVG
301 IDPFRLLQNS QVYSLIRPNE NPAHKSQLVW MACHSAAFED LRVLSFIKGT KVLPRGKLST
361 RGVQIASNEN METMESSTLE LRSRYWAIRT RSGGNTNQQR ASAGQISIQP TFSVQRNLPF
421 DRTTIMAAFN GNTEGRTSDM RTEIIRMMES ARPEDVSFQG RGVFELSDEK AASPIVPSFD
481 MSNEGSYFFG DNAEEYDN
***SEQ ID NO: 13 (PR*/*8*/*34 NP full length; 498aa)*** 
1 
61 
121 
181 
241 
301 
 
361 
421 
481 
***SEQ ID NO: 14 (Ox2R fragment)*** 
1 TKLEDSPPCR NWSSASELNE TQEPFLNPTD YDDEEFLRYL WREYLHPKEY EWVLIAGYII
61 VFVVALIGNV LVCVAVWKNH HMRTVTNYFI VNLSLADVLV TITCLPATLV VDITETWFFG
***SEQ ID NO: 15 (Ox1R fragment)*** 
1 MEPSATPGAQ MGVPPGSREP SPVPPDYEDE FLRYLWRDYL YPKQYEWVLI AAYVAVFVVA
61 LVGNTLVCLA VWRNHHMRTV TNYFIVNLSL ADVLVTAICL PASLLVDITE SWLFGHALCK
***SEQ ID NO: 16 (Orexin fragment)***
   MNLPSTKVSWAAV
***SEQ ID NO: 17 (fragment of SEQ ID NO: 1)***
   YDKEEIRRIWRQ
***SEQ ID NO: 18 (X-179A NP fragment)***
   LILYDKEEIRRIWRQ
***SEQ ID NO: 19 (X-181 NP fragment)***
   LILYDKEEMRRIWRQ
***SEQ ID NO: 20 (X-179A NP fragment)***
   VGKMIGGIGRFYIQM
***SEQ ID NO: 21***
   SGAAGAAVKGVGTMV
***SEQ ID NO: 22***
   EKATNPIVPSFDMSN
***SEQ ID NO: 23***
   IDPFKLLQNSQVVSL
***SEQ ID NO: 24***
   LILYDKEERRRRWRQ
***SEQ ID NO: 25***
   MNLPSTKVSWAAVTL
***SEQ ID NO: 26***
   MNLPSIKVSWAAVTL
***SEQ ID NO: 27***
   MNLPSMKVSWAAVTL
***SEQ ID NO: 28***
   LTVAAWSVKTSPLNM
***SEQ ID NO: 29***
   GAGNHAAGILTLGKR
***SEQ ID NO: 30***
   ASGNHAAGILTMGRR
***SEQ ID NO: 31***
   AMERNAGSGIIISDT
***SEQ ID NO: 32***
   ALNRGSGSGIITSDA
***SEQ ID NO: 33***
   ALSRGFGSGIITSNA

### REFERENCES

[1] Zaracostas J.; BMJ. 2011 Feb 9;342:d909
[2] Nohynek et al.. PLoS One 2012;7(3):e33536.
[3] Partinen et al. PLoS One 2012;7(3):e33723.
[4] Dauvilliers et al.; Sleep 2010;33(11):1428-1430.
[5] Szakacs et al.; Neurology 2013;80(14):1315-1321.
[6] http://www.who.int/influenza/vaccines/virus/recommendations/en/
[7] http://www.fda.gov/AdvisoryCommittees/CommitteesMeetingMaterials/BloodVaccinesandOther Biologics/VaccinesandRelatedBiologicalProductsAdvisoryCommittee/default.htm
[8] Siebold et al.; Proc Natl Acad Sci U S A 2004;101(7):1999-2004.
[9] Chaloupka et al 1996, Eur J Clin Microbiol Infect Dis. 1996 Feb;15(2):121-7
[10] Williams et al, 2012, Vaccine 30: 2475-2482.
[11] Getie-Kebtie et al., 2013, Influenza and Other Respiratory Viruses 7(4), 521-530.
[12] Kalandadze et al., 1996, J. Biol Chem. 271(33): 20156-20162.
[13] Justesen et al., 2009, Immunome Research 5: 2
[14] Kistner et al. (1998) Vaccine 16:960-8.
[15] Kistner et al. (1999) Dev Biol Stand 98:101-110.
[16] Bruhl et al. (2000) Vaccine 19:1149-58.
[17] WO2006/108846.
[18] Pau et al. (2001) Vaccine 19:2716-21.
[19] http://www.atcc.org/
[20] http://locus.umdnj.edu/
[21] WO97/37000.
[22] Brands et al. (1999) Dev Biol Stand 98:93-100.
[23] Halperin et al. (2002) Vaccine 20:1240-7.
[24] EP-A-1260581 (WO01/64846).
[25] WO2006/071563.
[26] WO2005/113758.
[27] Grachev et al. (1998) Biologicals;26(3): 175-93.
[28] WO97/37001
[29] WO02/28422.
[30] WO02/067983.
[31] WO02/074336.
[32] WO01/21151.
[33] WO02/097072.
[34] WO2005/113756.
[35] Huckriede et al. (2003) Methods Enzymol 373:74-91.
[36] Vaccines. (eds. Plotkins & Orenstein). 4th edition, 2004, ISBN: 0-7216-9688-0
[37] Treanor et al. (1996) J Infect Dis 173:1467-70.
[38] Keitel et al. (1996) Clin Diagn Lab Immunol 3:507-10.
[39] Herlocher et al. (2004) J Infect Dis 190(9):1627-30.
[40] Le et al. (2005) Nature 437(7062):1108.
[41] WO2008/068631.
[42] Rota et al. (1992) J Gen Virol 73:2737-42.
[43] GenBank sequence GI:325176.
[44] Gennaro (2000) Remington: The Science and Practice of Pharmacy. 20th ed, ISBN: 0683306472.
[45] Banzhoff (2000) Immunology Letters 71:91-96.
[46] Nony et al. (2001) Vaccine 27:3645-51.
[47] EP-B-0870508.
[48] US 5948410.
[49] WO2007/052163.
[50] WO2007/052061
[51] WO90/14837.
[52] Podda & Del Giudice (2003) Expert Rev Vaccines 2:197-203.
[53] Podda (2001) Vaccine 19: 2673-2680.
[54] Vaccine Design: The Subunit and Adjuvant Approach (eds. Powell & Newman) Plenum Press 1995 (ISBN 0-306-44867-X).
[55] Vaccine Adjuvants: Preparation Methods and Research Protocols (Volume 42 of Methods in Molecular Medicine series). ISBN: 1-59259-083-7. Ed. O'Hagan.
[56] Garçon et al. (2012) Expert Rev Vaccines 11:349-66.
[57] WO2008/043774.
[58] Allison & Byars (1992) Res Immunol 143:519-25.
[59] Hariharan et al. (1995) Cancer Res 55:3486-9.
[60] US-2007/014805.
[61] US-2007/0191314.
[62] Suli et al. (2004) Vaccine 22(25-26):3464-9.
[63] WO95/11700.
[64] US patent 6,080,725.
[65] WO2005/097181.
[66] WO2006/113373.
[67] Potter & Oxford (1979) Br Med Bull 35: 69-75.
[68] Greenbaum et al. (2004) Vaccine 22:2566-77.
[69] Zurbriggen et al. (2003) Expert Rev Vaccines 2:295-304.
[70] Piascik (2003) J Am Pharm Assoc (Wash DC). 43:728-30.
[71] Mann et al. (2004) Vaccine 22:2425-9.
[72] Halperin et al. (1979) Am J Public Health 69:1247-50.
[73] Herbert et al. (1979) J Infect Dis 140:234-8.
[74] Chen et al. (2003) Vaccine 21:2830-6.
[75] Needleman & Wunsch (1970) J. Mol. Biol. 48, 443-453.
[76] Rice et al. (2000) Trends Genet 16:276-277.
*[77]* Current Protocols in Molecular Biology (F.M. Ausubel et al., eds., 1987) Supplement 30.
[78] Smith & Waterman (1981) Adv. Appl. Math. 2: 482-489.
[79] Renfrey S, Watts A. Vaccine 1994;12(8):747-752.
[80] Carlson et al.; Arthritis Rheum 1999;42(12):2705-2709.
[81] Han et al.; Ann Neurol 2011;70(3):410-417.
[82] Ettinger et al.; J Immunol 2006;176(3):1988-1998.
[83] Bui et al.; Proc Natl Acad Sci U S A 2007;104(1):246-251
[84] Doucet et al.; J Gen Virol 2011;92(Pt 5):1162-1171.
[85] Yewdell et al.; Proc Natl Acad Sci U S A 1985;82(6):1785-1789.
[86] Willie et al.; Neuron 2003;38(5):715-730.
[87] Berkhoff et al.; J Virol 2005;79(17):11239-11246.
[88] Steinitz et al. Blood 2012; 119(17):4073-4082.

## Claims

1. An influenza vaccine composition comprising influenza virus A nucleoprotein wherein a fragment of said nucleoprotein equivalent to amino acids 106 to 126 of SEQ ID NO: 2 binds to an MHC class II receptor comprising HLA DQB1*0602 with a lower affinity than a peptide having the amino acid sequence shown in SEQ ID NO: 1, with the proviso that if all influenza A nucleoprotein in the composition comprises the amino acid sequence shown in SEQ ID NO: 12, then the vaccine composition is not based on strain A/California/7/2009 (H1N1)-derived strain NYMC X-181.

2. An influenza vaccine composition comprising influenza A virus nucleoprotein wherein none of said nucleoprotein comprises a fragment equivalent to amino acids 106 to 126 of SEQ ID NO: 2 which binds to an MHC class II receptor comprising HLA DQB 1*0602 with an equal or higher affinity than a peptide having the amino acid sequence shown in SEQ ID NO:1, with the proviso that if all influenza A nucleoprotein in the composition comprises the sequence shown in SEQ ID NO: 12, then the vaccine composition is not based on strain A/California/7/2009 (H1N1)-derived strain NYMC X-181.

3. An influenza vaccine composition according to claim 1 or claim 2 wherein not all of the nucleoprotein in the composition comprises the amino sequence shown in SEQ ID NO: 12.

4. An influenza vaccine composition according to claim 3 wherein not all of the nucleoprotein in the composition comprises the amino sequence shown in SEQ ID NO: 3.

5. An influenza vaccine composition comprising influenza virus A nucleoprotein wherein said nucleoprotein does not have an isoleucine residue at a position corresponding to amino acid 116 of the nucleoprotein amino acid sequence shown in SEQ ID NO: 2, with the proviso that if all influenza A nucleoprotein in the composition comprises the amino acid sequence shown in SEQ ID NO: 12, then the vaccine composition is not based on strain A/California/7/2009 (H1N1)-derived strain NYMC X-181.

6. An influenza vaccine composition comprising influenza virus A nucleoprotein wherein said nucleoprotein does not have an isoleucine at a position corresponding to amino acid 116 of the nucleoprotein amino acid sequence shown in SEQ ID NO: 2, with the proviso that if all of the nucleoprotein comprises a methionine at a position corresponding to amino acid 116 of the nucleoprotein amino acid sequence shown in SEQ ID NO: 2 then said nucleoprotein does not have the sequence shown as SEQ ID NO: 12.

7. An influenza vaccine composition comprising influenza virus A nucleoprotein wherein said nucleoprotein does not have an isoleucine or a methionine residue at a position corresponding to amino acid 116 of the nucleoprotein amino acid sequence shown in SEQ ID NO: 2.

8. An influenza vaccine composition comprising influenza virus A nucleoprotein wherein said nucleoprotein does not comprise the amino acid sequence shown in SEQ ID NO: 2, SEQ ID NO: 12, or SEQ ID NO: 13.

9. An influenza vaccine composition comprising influenza virus A nucleoprotein wherein the nucleoprotein has been modified to reduce or abolish its binding to an MHC class II receptor comprising HLA DQB1*0602 as compared with the unmodified nucleoprotein.

10. An influenza vaccine composition comprising influenza virus nucleoprotein wherein (i) the composition is a split virion vaccine and the amount of nucleoprotein present is less than 3µg nucleoprotein per 10 µg of hemagglutinin or (ii) the composition is a subunit vaccine and the amount of nucleoprotein present is less than 0.5 µg nucleoprotein per 10 µg of hemagglutinin.

11. A vaccine composition according to any one of the preceding claims further comprising an adjuvant.

12. A vaccine composition according to claim 11 wherein the adjuvant is an oil-in-water emulsion.

13. A vaccine composition according to claim 12 wherein the adjuvant further comprises tocopherol.

14. A vaccine composition according to any one of the preceding claims further comprising Triton or Tween, or a combination thereof.

15. A vaccine composition according to any one of the preceding claims which is a vaccine against one or more pandemic influenza strains.

16. A vaccine composition according to any one of claims 11 to 14 which is a monovalent vaccine composition.

17. A vaccine composition according to any one of the preceding claims which is a split virion vaccine.

18. An adjuvanted split influenza vaccine wherein the vaccine comprises antigens from at least 4 different influenza viruses and nucleoprotein from at least one influenza A virus having the amino acid sequence shown in SEQ ID NO: 2, **characterized in that** the adjuvant is an oil-in-water emulsion adjuvant which does not contain an additional immunostimulating agent, and whereby the composition contains Triton.

19. A vaccine or a vaccine composition according to any one of the preceding claims for use in the pediatric population (0-36 months) and/or the adolescent population (4-19 years) and/or in subjects with a genetic predisposition to develop an autoimmune disease in connection with flu vaccination.

20. A method of testing an influenza A virus for suitability for vaccine production, comprising a step of determining whether the influenza virus's nucleoprotein, or a fragment thereof, can bind to HLA DQB 1*0602 with lower affinity under the same conditions compared to nucleoprotein from H1N1 strain X-179A; wherein the influenza virus is suitable for vaccine production if its nucleoprotein can bind to HLA DQB1*0602 with lower affinity under the same conditions compared to nucleoprotein from strain X-179A.
